# EUROPEAN PATENT APPLICATION

(11) **EP 4 847 028 A1**
(43) Date of publication of application: **02.12.2026**
(21) Application number: 25179369.1
(22) Date of filing: 28.05.2025
(51) Int. Cl.: A61K 35/74, A23L 33/135, A61K 35/741, A61K 35/744, A61P 29/00, A61P 37/06, A61K 35/00, C12R 1/25

(54) **LACTIPLANTIBACILLUS AND/OR LACTICASEIBACILLUS COMPOSITION AND USES THEREOF**

(71) Applicant: Probi AB, 223 70 Lund (SE)
(72) Inventor: RANGEL, Ignacio, 703 74 Örebro (SE); CHATZOPOULOU, Myrto S., 703 67 Örebro (SE); ÖNNING, Gunilla, 223 70 Lund (SE)
(74) Representative: Potter Clarkson

(57) **Abstract**

The disclosure relates among others to methods and uses of at least one strain of *Lactiplantibacillus plantarum* and/or *Lacticaseibacillus paracasei* for increasing and/or promoting biofilm formation in the gastrointestinal tract of a subject. The disclosure further relates to methods and uses of at least one strain of *Lactiplantibacillus plantarum* for increasing growth of at least one strain of *Lactiplantibacillus plantarum* in the gastrointestinal tract of a subject, said subject having an elevated expression of one or more pro-inflammatory cytokines.

## Description

### TECHNICAL FIELD

Among others the disclosure relates to methods and uses of at least one strain of *Lactiplantibacillus plantarum* and/or *Lacticaseibacillus paracasei* for increasing and/or promoting biofilm formation in the gastrointestinal tract of a subject. The disclosure further relates to methods and uses of at least one strain of *Lactiplantibacillus plantarum* for increasing growth of at least one strain of *Lactiplantibacillus plantarum* in the gastrointestinal tract of a subject, said subject having an elevated expression of one or more pro-inflammatory cytokines.

### BACKGROUND

The intestinal microbiota is composed of more than one thousand bacterial species, and it is involved in several gastrointestinal processes, such as the uptake of nutrients, production of essential metabolites and neurotransmitters, development of the immune system, and inflammatory responses. During chronic inflammation, gastrointestinal homeostasis is disrupted with detrimental consequences on both the host, but also the composition and function of the gastrointestinal microbiome.

Gastrointestinal homeostasis is defined by a proper coordination of pro-inflammatory responses against dietary antigens, pathogens, or their metabolites with nutrient absorption and tolerance for non-invasive, commensal bacteria. However, the homeostatic state can be disrupted due to numerous factors either intrinsic (e.g., genetics, gender, age) or extrinsic (e.g., diet, exposure to pollutants, smoking, infections), which often correlate with unfavourable alterations in the composition and function of the gastrointestinal microbiota, a phenomenon known as dysbiosis. Dysbiosis has been found to be a common denominator in several diseases, such as inflammatory bowel disease (e.g., Crohn's disease, ulcerative colitis, microscopic colitis), irritable bowel syndrome, metabolic syndromes, allergies, neurodegenerative and cardiovascular diseases.

Differential gene expression of pro-inflammatory cytokines is an important component of gastrointestinal pathology, especially in inflammatory bowel disease (IBD), but also irritable bowel syndrome (IBS). Over-expression of pro-inflammatory mediators, interferon gamma (IFN-γ), interleukin-6 (IL-6), interleukin-1β (IL- 1β), tumor necrosis factor (TNF-α), and chemokine CXCL8 (or interleukin-8, IL-8) is characteristic of an inflamed gastrointestinal tract and their interactions with the intestinal epithelium, immune cells and the enteric nervous system (ENS) have been well studies.

Biofilms are communities of microbes embedded in matrices composed of extracellular polymeric substance (EPS) and have been implicated in both the healthy microbiome and disease states. Biofilms reduce the effectiveness of both polymorphonuclear neutrophils and macrophages. In addition, in the presence of these cells, biofilm formation is actively enhanced, and components of host immune cells are assimilated into the EPS matrix. While pathogenic biofilms contribute to chronic inflammation, probiotic *Lacticaseibacillus* biofilms cause a negligible immune response and, in states of inflammation, exhibit robust anti-inflammatory properties.

Based on the above it is clear that there is a need in the art to identify ways of increasing or promoting probiotic biofilm formation in the gastrointestinal tract of subjects suffering from excessive immune responses in the gastrointestinal tract.

### SUMMARY

The inventors have surprisingly found that exposure to pro-inflammatory cytokines (TNF-α, IL-1β, IL-6, IFN-γ and CXCL8) can significantly increase biofilm production of the probiotic bacterial strains *Lactiplantibacillus plantarum* DSM 9843, *Lactiplantibacillus plantarum* DSM 15312 and *Lacticaseibacillus paracasei* DSM 13434. Specifically, biofilm formation was observed in these strains when stimulated with pro-inflammatory cytokines selected from the group consisting of TNF-α, IL-1β, IL-6, IFN-γ and CXCL8 (See Example 1). These findings suggest that pro-inflammatory cytokines can influence the phenotypic characteristics of probiotic strains, promoting their biofilm formation. These probiotic biofilms can colonize the gastrointestinal tract, forming an anti-inflammatory barrier that protects the gastrointestinal tract from both low- and high-grade inflammation. In addition to increased biofilm formation the inventors also found that exposure to pro-inflammatory cytokines (TNF-α, IL-1β, IL-6, IFN-γ and CXCL8) significantly increased growth of *Lactiplantibacillus plantarum* DSM 9843 and *Lactiplantibacillus plantarum* DSM 15312 (See Example 1).

Thus, an object of the disclosure relates to the provision of methods and uses of *Lactiplantibacillus plantarum* and/or *Lacticaseibacillus paracasei* for increasing and/or promoting biofilm formation in the gastrointestinal tract of a subject. A further object of the disclosure relates to methods and uses of at least one strain of *Lactiplantibacillus plantarum* for increasing growth of at least one strain of *Lactiplantibacillus plantarum* in the gastrointestinal tract of a subject, said subject having an elevated expression of one or more pro-inflammatory cytokines.

Therefore, a first aspect of the disclosure relates to a method for increasing and/or promoting biofilm formation in the gastrointestinal tract of a subject, comprising administering to a subject in need thereof, an effective dose of at least one strain of *Lactiplantibacillus plantarum* and/or *Lacticaseibacillus paracasei.*

A second aspect of the disclosure disclosure relates to at least one strain of *Lactiplantibacillus plantarum* and/or *Lacticaseibacillus paracasei* for use in increasing and/or promoting biofilm formation in the gastrointestinal tract of a subject.

A third aspect of the disclosure relates to the use of at least one strain of *Lactiplantibacillus plantarum* and/or *Lacticaseibacillus paracasei* for increasing and/or promoting biofilm formation in the gastrointestinal tract of a subject.

A fourth aspect of the disclosure relates to a non-therapeutic use of at least one strain of *Lactiplantibacillus plantarum* and/or *Lacticaseibacillus paracasei* for increasing and/or promoting biofilm formation in the gastrointestinal tract of a subject.

A fifth aspect of the disclosure relates to the use of at least one strain of *Lactiplantibacillus plantarum* and/or *Lacticaseibacillus paracasei* in the manufacture of a medicament for increasing and/or promoting biofilm formation in the gastrointestinal tract of a subject.

A sixth aspect of the disclosure relates to the use of at least one strain of *Lactiplantibacillus plantarum* and/or *Lacticaseibacillus paracasei* in the manufacture of a supplement for increasing and/or promoting biofilm formation in the gastrointestinal tract of a subject.

A seventh aspect of the disclosure relates to a method for reducing, preventing and/or treating inflammation in the gastrointestinal tract of a subject, comprising administering to a subject in need thereof, an effective dose of at least one strain of *Lactiplantibacillus plantarum* and/or *Lacticaseibacillus paracasei.*

An eighth aspect of the disclosure relates to at least one strain of *Lactiplantibacillus plantarum* and/or *Lacticaseibacillus paracasei* for use in reducing, preventing and/or treating inflammation in the gastrointestinal tract of a subject.

A ninth aspect of the disclosure relates toto the use of at least one strain of *Lactiplantibacillus plantarum* and/or *Lacticaseibacillus paracasei* for reducing, preventing and/or treating inflammation in the gastrointestinal tract of a subject.

A tenth aspect of the disclosure relates to the use of at least one strain of *Lactiplantibacillus plantarum* and/or *Lacticaseibacillus paracasei* in the manufacture of a medicament for reducing, preventing and/or treating inflammation in the gastrointestinal tract of a subject.

An eleventh aspect of the disclosure relates to a method for reducing, preventing and/or treating intestinal barrier dysfunction in a subject, comprising administering to a subject in need thereof, an effective dose of at least one strain of *Lactiplantibacillus plantarum* and/or *Lacticaseibacillus paracasei.*

A twelfth aspect of the disclosure relates to at least one strain of *Lactiplantibacillus plantarum* and/or *Lacticaseibacillus paracasei* for use in reducing, preventing and/or treating intestinal barrier dysfunction in a subject.

A thirteenth aspect of the disclosure relates to the use of at least one strain of *Lactiplantibacillus plantarum* and/or *Lacticaseibacillus paracasei* for reducing, preventing and/or treating intestinal barrier dysfunction in a subject.

A fourteenth aspect of the disclosure relates to the use of at least one strain of *Lactiplantibacillus plantarum* and/or *Lacticaseibacillus paracasei* in the manufacture of a medicament for reducing, preventing and/or treating intestinal barrier dysfunction in a subject.

A fifteenth aspect of the disclosure relates to a method for improving and/or supporting gastrointestinal health in a subject, comprising administering to a subject in need thereof, an effective dose of at least one strain of *Lactiplantibacillus plantarum* and/or *Lacticaseibacillus paracasei.*

A sixteenth aspect of the disclosure relates to at least one strain of *Lactiplantibacillus plantarum* and/or *Lacticaseibacillus paracasei* for improving and/or supporting gastrointestinal health in a subject.

A seventeenth aspect of the disclosure relates to the use of at least one strain of *Lactiplantibacillus plantarum* and/or *Lacticaseibacillus paracasei* for improving and/or supporting gastrointestinal health in a subject.

An eighteenth aspect of the disclosure relates to the use of at least one strain of *Lactiplantibacillus plantarum* and/or *Lacticaseibacillus paracasei* in the manufacture of a medicament for improving and/or supporting gastrointestinal health in a subject.

A nineteenth aspect of the disclosure relates to a method for improving and/or supporting gastrointestinal homeostasis in a subject, comprising administering to a subject in need thereof, an effective dose of at least one strain of *Lactiplantibacillus plantarum* and/or *Lacticaseibacillus paracasei.*

A twentieth aspect of the disclosure relates to at least one strain of *Lactiplantibacillus plantarum* and/or *Lacticaseibacillus paracasei* for use in improving and/or supporting gastrointestinal homeostasis in a subject.

A twenty-first aspect of the disclosure relates to the use of at least one strain of *Lactiplantibacillus plantarum* and/or *Lacticaseibacillus paracasei* for improving and/or supporting gastrointestinal homeostasis in a subject.

A twenty-second aspect of the disclosure relates to the use of at least one strain of *Lactiplantibacillus plantarum* and/or *Lacticaseibacillus paracasei* in the manufacture of a medicament for improving and/or supporting gastrointestinal homeostasis in a subject.

A twenty-third aspect of the disclosure relates to a method for supporting and/or maintaining gastrointestinal endothelial function in a subject, comprising administering to a subject in need thereof, an effective dose of at least one strain of *Lactiplantibacillus plantarum* and/or *Lacticaseibacillus paracasei.*

A twenty-fourth aspect of the disclosure relates to at least one strain of *Lactiplantibacillus plantarum* and/or *Lacticaseibacillus paracasei* for use in supporting and/or maintaining gastrointestinal endothelial function in a subject.

A twenty-fifth aspect of the disclosure relates to the use of at least one strain of *Lactiplantibacillus plantarum* and/or *Lacticaseibacillus paracasei* for supporting and/or maintaining gastrointestinal endothelial function in a subject.

A twenty-sixth aspect of the disclosure relates to the use of at least one strain of *Lactiplantibacillus plantarum* and/or *Lacticaseibacillus paracasei* in the manufacture of a medicament for supporting and/or maintaining gastrointestinal endothelial function in a subject.

A twenty-seventh aspect of the disclosure relates to a method for improving and/or supporting metabolic health in a subject, comprising administering to a subject in need thereof, an effective dose of at least one strain of *Lactiplantibacillus plantarum* and/or *Lacticaseibacillus paracasei.*

A twenty-eighth aspect of the disclosure relates to at least one strain of *Lactiplantibacillus plantarum* and/or *Lacticaseibacillus paracasei* for use in improving and/or supporting metabolic health in a subject.

A twenty-ninth aspect of the disclosure relates to the use of at least one strain of *Lactiplantibacillus plantarum* and/or *Lacticaseibacillus paracasei* for improving and/or supporting metabolic health in a subject.

A thirtieth aspect of the disclosure relates to the use of at least one strain of *Lactiplantibacillus plantarum* and/or *Lacticaseibacillus paracasei* in the manufacture of a medicament for improving and/or supporting metabolic health in a subject.

A thirty-first aspect of the disclosure relates to a method preventing and/or treating a gastrointestinal condition, a gastrointestinal disorder relating to GI health or to metabolic syndrome and/or a metabolic disease in a subject, comprising administering to a subject in need thereof, an effective dose of at least one strain of *Lactiplantibacillus plantarum* and/or *Lacticaseibacillus paracasei.*

A thirty-second aspect of the disclosure relates to at least one strain of *Lactiplantibacillus plantarum* and/or *Lacticaseibacillus paracasei* for use in preventing and/or treating a gastrointestinal condition, a gastrointestinal disorder relating to GI health or to metabolic syndrome and/or a metabolic disease in a subject.

A thirty-third aspect of the disclosure relates to the use of at least one strain of *Lactiplantibacillus plantarum* and/or *Lacticaseibacillus paracasei* for preventing and/or treating a gastrointestinal condition, a gastrointestinal disorder relating to GI health or to metabolic syndrome and/or a metabolic disease in a subject.

A thirty-fourth aspect of the disclosure relates to the use of at least one strain of *Lactiplantibacillus plantarum* and/or *Lacticaseibacillus paracasei* in the manufacture of a medicament for preventing and/or treating a gastrointestinal condition, a gastrointestinal disorder relating to GI health or to metabolic syndrome and/or a metabolic disease in subject.

A thirty-fifth aspect of the disclosure relates to a method for reducing, preventing and/or treating systemic inflammation in a subject, comprising administering to a subject in need thereof, an effective dose of at least one strain of *Lactiplantibacillus plantarum* and/or *Lacticaseibacillus paracasei.*

A thirty-sixth aspect of the disclosure relates to at least one strain of *Lactiplantibacillus plantarum* and/or *Lacticaseibacillus paracasei* for use in reducing, preventing and/or treating systemic inflammation in a subject.

A thirty-seventh aspect of the disclosure relates to the use of at least one strain of *Lactiplantibacillus plantarum* and/or *Lacticaseibacillus paracasei* for reducing, preventing and/or treating systemic inflammation in a subject.

A thirty-eighth aspect of the disclosure relates to the use of at least one strain of *Lactiplantibacillus plantarum* and/or *Lacticaseibacillus paracasei* in the manufacture of a medicament reducing, preventing and/or treating systemic inflammation in a subject.

A thirty-ninth aspect of the disclosure relates to a method for increasing growth of at least one strain of *Lactiplantibacillus plantarum* in the gastrointestinal tract a subject having an elevated expression of one or more pro-inflammatory cytokines, comprising administering to a subject in need thereof, an effective dose of at least one strain of *Lactiplantibacillus plantarum.*

A fortieth aspect of the disclosure relates to at least one strain of *Lactiplantibacillus plantarum* for use in increasing growth of at least one strain of *Lactiplantibacillus plantarum* in the gastrointestinal tract of a subject having an elevated expression of one or more pro-inflammatory cytokines.

A forty-first aspect of the disclosure relates to the use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A forty-second aspect of the disclosure relates to a non-therapeutic use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A forty-third aspect of the disclosure relates to the use of at least one strain of *Lactiplantibacillus plantarum* in the manufacture of a medicament for increasing growth of at least one strain of *Lactiplantibacillus plantarum* in the gastrointestinal tract of a subject having an elevated expression of one or more pro-inflammatory cytokines.

A forty-fourth aspect of the disclosure relates to the use of at least one strain of *Lactiplantibacillus plantarum* in the manufacture of a supplement for increasing growth of at least one strain of *Lactiplantibacillus plantarum* in the gastrointestinal tract of a subject having an elevated expression of one or more pro-inflammatory cytokines.

The present invention will in the following be described in more detail.

### BRIEF DESCRIPTION OF THE DRAWINGS

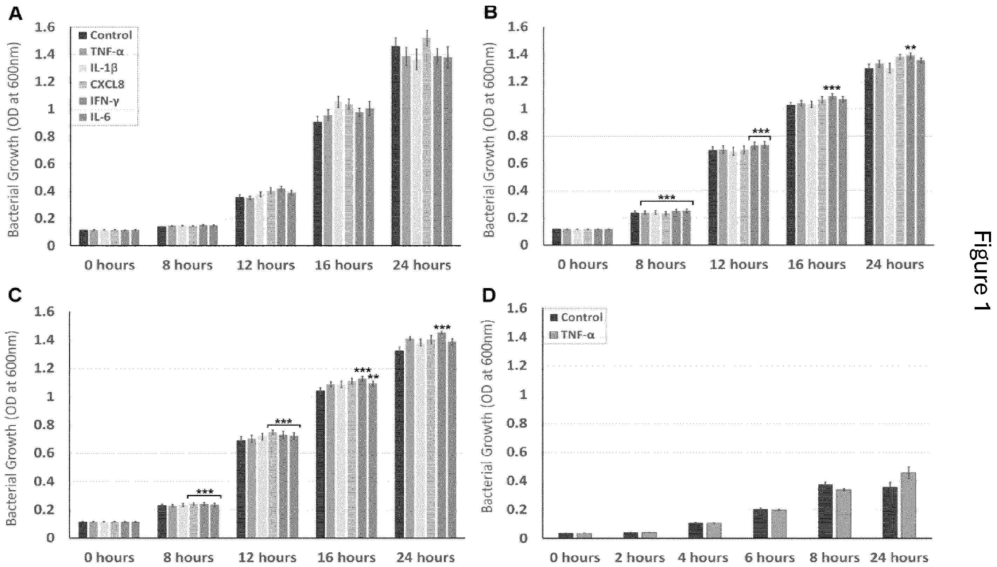

**Figure 1****.** Shows bacterial growth with or without TNF-α, IL-1β, CXCL8, IFN-γ, or IL-6 (40 ng/mL) incubation for *Lacticaseibacillus paracasei* DSM 13434 (A), *Lactiplantibacillus plantarum* DSM 15312 (B), *Lactiplantibacillus plantarum* DSM 9843 (C), and with or without TNF-α (40 ng/mL) incubation for E. coli ATCC 8739 (D). Unstimulated bacteria are referred to as "Control". Data are presented as mean ± SEM of n = 7 independent experiments for A, B & C, and n=3 for D. Statistical significance is denoted with asterisks: **p ≤ 0.01 and ***p ≤ 0.001 in comparison with the control.

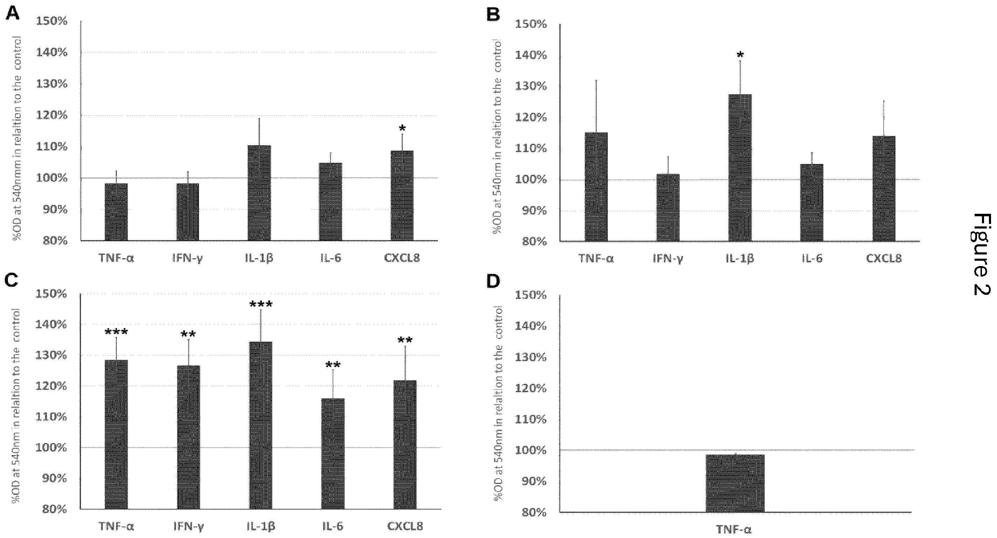

**Figure 2****.** Shows bacterial biofilm formation with or without TNF-α, IL-1β, CXCL8, IFN-γ, or IL-6 (40 ng/mL) incubation for *Lacticaseibacillus paracasei* DSM 13434 (A), *Lactiplantibacillus plantarum* DSM 15312 (B), *Lactiplantibacillus plantarum* DSM 9843 (C), and with or without TNF-α (40 ng/mL) incubation for E. coli ATCC 8739 (D). The black line represents the unstimulated bacteria at 100%. Data are presented as mean ± SEM of n = 4 independent experiments for A, B & C, and n=3 for D. Statistical significance is denoted with asterisks: *p ≤ 0.05, **p ≤ 0.01, and ***p ≤ 0.001 in comparison with the control.

### DETAILED DESCRIPTION

The disclosure relates to a method for increasing and/or promoting biofilm formation in the gastrointestinal tract of a subject. In particular, the disclosure relates to the provision of methods and uses of *Lactiplantibacillus plantarum* and/or *Lacticaseibacillus paracasei* for increasing and/or promoting biofilm formation in the gastrointestinal tract of a subject. The disclosure also relates to methods and uses of at least one strain of *Lactiplantibacillus plantarum* for increasing growth of at least one strain of *Lactiplantibacillus plantarum* in the gastrointestinal tract of a subject, said subject having an elevated expression of one or more pro-inflammatory cytokines.

The inventors of the present invention have surprisingly succeeded in developing a method for increasing and/or promoting biofilm formation. Experimental evidence has successfully shown that stimulating probiotic strains of the *Lactiplantibacillus plantarum* and *Lacticaseibacillus paracasei* species with pro-inflammatory cytokines results in increased biofilm formation while stimulating probiotic strains of the *Lactiplantibacillus plantarum* with pro-inflammatory cytokines results in enhanced growth of *Lactiplantibacillus plantarum.*

Therefore, a first aspect of the disclosure relates to a method for increasing and/or promoting biofilm formation in the gastrointestinal tract of a subject, comprising administering to a subject in need thereof, an effective dose of at least one strain of *Lactiplantibacillus plantarum* and/or *Lacticaseibacillus paracasei.*

By "increasing and/or promoting" we include the meaning of a method which gives rise to an effect in a subject of facilitating and/or supporting conditions that enables probiotic bacteria to thrive, colonize the gastrointestinal tract of a subject and form biofilm.

By "biofilm formation" we include the meaning of a process involving bacteria that adhere to the surfaces within the gastrointestinal tract and which produces extracellular polymeric substances (EPS). Biofilm formation in the gastrointestinal tract involves bacteria adhering to the gastrointestinal tract lining and creating a protective layer. This biofilm helps the bacteria to thrive by shielding them from harmful substances and facilitating nutrient exchange. Biofilm plays a crucial role in maintaining gastrointestinal homeostasis and gastrointestinal health, enhancing immune function, and preventing the colonization of pathogenic bacteria. Biofilm formation may be measured *in vitro* by a biofilm formation assay, as described in Example 1, where bacteria are incubated in a microtiter plate for 24 hours in the presence of cytokines. The biofilm formation may be quantified by optic density at 540nm using microplate reader (Cytation 3, Biotek Inc).

### Biofilm formation in the gastrointestinal tract

The formation of biofilms in the gastrointestinal tract is initiated by the attachment of microorganisms to a mucosal surface in the gastrointestinal tract. This attachment is facilitated by adhesins, surface proteins that enable microbes to adhere to the epithelial cells lining the gastrointestinal tract. Once attached, the microorganisms produce EPS, a matrix composed of polysaccharides, proteins, and nucleic acids. This matrix anchors the microorganisms to the surface and provides structural integrity to the developing biofilm. As the biofilm matures, it undergoes developmental stages characterized by microbial proliferation and the recruitment of additional species. This results in a diverse and stratified community of microbes, where different microbial species occupy distinct niches. The spatial organization within the biofilm allows for metabolic cooperation and communication among the microbes. Quorum sensing, a cell-to-cell communication mechanism, regulates biofilm development by coordinating gene expression in response to population density. It plays a crucial role in the maturation and maintenance of the biofilm. Factors such as pH, nutrient availability, and host immune responses influence biofilm formation. These factors can modulate the expression of genes involved in adhesion, EPS production, and microbial interactions, shaping the biofilm's structure and function. As disclosed in Example 1, exposure of *Lactiplantibacillus plantarum* and/or *Lacticaseibacillus paracasei* to pro-inflammatory cytokines selected from the group consisting of TNF-α, IL-1β, IL-6, IFN-γ and CXCL8 may increase biofilm production. It is to be understood that the biofilm produced is produced by *Lactiplantibacillus plantarum* and/or *Lacticaseibacillus paracasei.*

The second aspect of the disclosure relates to at least one strain of *Lactiplantibacillus plantarum* and/or *Lacticaseibacillus paracasei* for use in increasing and/or promoting biofilm formation in the gastrointestinal tract of a subject.

A third aspect of the disclosure relates to the use of at least one strain of *Lactiplantibacillus plantarum* and/or *Lacticaseibacillus paracasei* for increasing and/or promoting biofilm formation in the gastrointestinal tract of a subject.

A fourth aspect of the disclosure relates to a non-therapeutic use of at least one strain of *Lactiplantibacillus plantarum* and/or *Lacticaseibacillus paracasei* for increasing and/or promoting biofilm formation in the gastrointestinal tract of a subject.

Biofilm formation in the gastrointestinal tract of a subject is considered to be increased and/or promoted when the amount of biofilm in the gastrointestinal tract of a subject administered at least one strain of *Lactiplantibacillus plantarum* and/or *Lacticaseibacillus paracasei* exceeds the amount of biofilm formed in the gastrointestinal tract of a subject who has not been administered the at least one strain of *Lactiplantibacillus plantarum* and/or *Lacticaseibacillus paracasei.*

By "gastrointestinal tract" we include the meaning of the gastrointestinal (GI) tract including the stomach, small intestine and large intestine. In the present context the terms "gut" and "gastrointestinal tract" are used herein interchangeably.

By "non-therapeutic use" we include the meaning of non-medical use, which is to be understood as not relating to, being, or providing therapy.

A fifth aspect of the disclosure relates to the use of at least one strain of *Lactiplantibacillus plantarum* and/or *Lacticaseibacillus paracasei* in the manufacture of a medicament for increasing and/or promoting biofilm formation in the gastrointestinal tract of a subject.

A sixth aspect of the disclosure relates to the use of at least one strain of *Lactiplantibacillus plantarum* and/or *Lacticaseibacillus paracasei* in the manufacture of a supplement for increasing and/or promoting biofilm formation in the gastrointestinal tract of a subject.

### Inflammation in the gastrointestinal tract

Inflammation in the gastrointestinal tract may also be referred to as gastrointestinal inflammation, can be caused by various factors, including infections, autoimmune disorders, and chronic diseases like inflammatory bowel disease (IBD). Common symptoms include abdominal pain, diarrhea, fatigue, and weight loss. IBDs, such as Crohn's disease and ulcerative colitis, are chronic conditions with unknown exact causes. However, they are believed to result from a combination of immunological, genetic, and environmental factors, as well as changes in the gastrointestinal microbiota.

In patients with IBD, the gastrointestinal tract becomes more permeable, a condition known as intestinal leakiness. This is due to microbial imbalance (dysbiosis), inflammation, and alterations in tight junctions, which are proteins that seal cells together. The gastrointestinal tract microbiota of IBD patients often contains more pro-inflammatory bacteria like adherent-invasive *Escherichia coli* and mucolytic bacteria such as *Ruminococcus gnavus* and *Ruminococcus torques,* along with reduced microbial diversity. During active phases of IBD, the presence of enterotoxigenic *Bacteroides fragilis,* which produces metalloproteases, increases, leading to inflammatory diarrhea. The inflammatory response in the gastrointestinal tract is driven by altered levels of cytokines such as TNF-α, TGF-β, IL-17, IL-22, and IL-23, and mutations in the NOD2 gene. These mutations contribute to the enhanced inflammatory response in the gastrointestinal tract mucosa. In Crohn's disease, NOD2 mutations affect the expression of defensin genes, impairing antimicrobial responses and allowing bacteria to cross the gastrointestinal tract lining.

Metabolic changes in IBD patients include reduced levels of short-chain fatty acids and increased levels of polyunsaturated fatty acids, including arachidonate, which have pro-inflammatory effects. Additionally, bile acid conversion is reduced. Dysregulation of tight junctions is also observed, with TNF-α and IL-13 disrupting these junctions and promoting cell death in the gastrointestinal tract lining, particularly in ulcerative colitis.

Modulating gastrointestinal tract microbiota through dietary supplementation of probiotic strains of *Lactiplantibacillus* and/or *Lacticaseibacillus* has been shown to be an effective strategy to prevent inflammatory diseases and disorders such as, IBD, metabolic syndrome, and chronic infections in patients.

Therefore, it may be advantageous to provide a method for reducing, preventing and/or treating inflammation in the gastrointestinal tract of a subject, comprising administering to a subject in need thereof, an effective dose of at least one strain of *Lactiplantibacillus plantarum* and/or *Lacticaseibacillus paracasei.*

Thus, a seventh aspect of the disclosure relates to a method for reducing, preventing and/or treating inflammation in the gastrointestinal tract of a subject, comprising administering to a subject in need thereof, an effective dose of at least one strain of *Lactiplantibacillus plantarum* and/or *Lacticaseibacillus paracasei.*

By "reducing" we include the meaning of a use which gives rise to an effect in a subject of preventing, delaying, protecting against, reducing the severity of and/or removing, one or more effects, symptoms and/or other markers associated with a disorder, disease or condition.

By "preventing" we include the meaning that the event, effect or condition being prevented is protected against, delayed, reduced (e.g. reduced in severity), blocked from occurring, or made to cease. Such prevention typically takes place before the event occurs or the effect or condition is manifest, but it will be appreciated that it can also mean to prevent further occurrence of the same kind of event. It will also be appreciated that such terms may include the meaning that an event or condition is maintained in the current state without becoming worse or developing further.

By "treating" we include the meaning that the disease or condition being treated is ameliorated, reduced in severity, removed, blocked from occurring further, protected against occurring further, delayed and/or made to cease. The terms "treat", "treating" and "treatment" also include the meaning of delaying, protecting against, reducing the severity of and/or removing, one or more symptoms, effects, indications and/or other markers associated with a given disease or condition. Where one of these terms is used in the context of a disease or condition, such treatment typically takes place after the disease or condition is manifest. It will also be appreciated that such terms may include the meaning that a disease or condition is maintained in the current state without becoming worse or developing further.

By" "inflammation in the gastrointestinal tract we include the meaning of chronic inflammation in the gastrointestinal tract. Chronic inflammation in the gastrointestinal tract is a prolonged inflammatory response within the gastrointestinal tract. This condition can result from various factors, including autoimmune disorders like Crohn's disease and ulcerative colitis. It involves persistent activation of the immune system, leading to damage of the gastrointestinal tract lining, which can cause symptoms such as abdominal pain, diarrhea, fatigue, and weight loss. Inflammation can be measured in the blood by an elevated C-Reactive Protein (CRP) level >3 mg/L. CRP in blood can be measured by immunoturbidimetry.

An eighth aspect of the disclosure relates to at least one strain of *Lactiplantibacillus plantarum* and/or *Lacticaseibacillus paracasei* for use in reducing, preventing and/or treating inflammation in the gastrointestinal tract of a subject.

A ninth aspect of the disclosure relates to the use of at least one strain of *Lactiplantibacillus plantarum* and/or *Lacticaseibacillus paracasei* for reducing, preventing and/or treating inflammation in the gastrointestinal tract of a subject.

A tenth aspect of the disclosure relates to the use of at least one strain of *Lactiplantibacillus plantarum* and/or *Lacticaseibacillus paracasei* in the manufacture of a medicament for reducing, preventing and/or treating inflammation in the gastrointestinal tract of a subject.

An eleventh aspect of the disclosure relates to a method for reducing, preventing and/or treating intestinal barrier dysfunction in a subject, comprising administering to a subject in need thereof, an effective dose of at least one strain of *Lactiplantibacillus plantarum* and/or *Lacticaseibacillus paracasei.*

### Intestinal barrier

The intestinal barrier is a crucial component of the digestive system, responsible for maintaining the balance between absorbing nutrients and protecting the body from harmful substances, pathogens and infection. It consists of several elements, including physical, biochemical, and immune components. The physical barrier consists of the intestinal epithelium which forms the foremost layer, consisting of tightly packed epithelial cells. These cells are connected by junctional complexes that seal the space between them, preventing the uncontrolled passage of substances. The mucosal surface of the gastrointestinal tract is covered by mucus, a substance composed mainly of water (normally >98%) and the proteins MUC2 and MUCA5C in the stomach, which are produced by goblet cells. The mucus forms a protective layer that separates the bulk of luminal contents from the epithelial cells. It prevents large particles and microorganisms from contacting the epithelial layer while allowing small molecules to pass.

The biochemical barrier consists of various molecules secreted into the lumen reinforce the barrier function. These include antimicrobial peptides and enzymes that help neutralize harmful substances. The immune barrier consists of immune cells located below the epithelial layer provide additional protection. They detect and respond to pathogens, preventing infections and maintaining gastrointestinal health. Additionally, the gastrointestinal tract microbiota which consists of commensal microbial species inhabiting the gastrointestinal tract also play a role in the intestinal barrier. They stimulate epithelial cell proliferation, produce short-chain fatty acids, and help maintain the integrity of the barrier.

Taken together, the intestinal barrier ensures that undesirable luminal contents, such as toxins and pathogens, are contained within the intestine while allowing the absorption of beneficial nutrients. Its integrity is vital for overall health, as intestinal barrier dysfunction can lead to various conditions, including infections, irritable bowel syndrome (IBS), inflammatory bowel disease (IBD), and metabolic disorders.

### Intestinal barrier dysfunction

When the intestinal barrier is compromised, such as by severe mucosal damage, its permeability increases. This allows bacteria, their fragments, or products to pass through the lumen. An altered intestinal barrier can lead to increased permeability and an inflammatory response. The gastrointestinal tract microbiota can influence this process, leading to conditions known as leaky gastrointestinal tract syndrome and dysbiosis. Both conditions are involved in the development of various gastrointestinal and systemic disorders.

Probiotics, such as lactic acid bacteria, have been extensively studied for their immunomodulatory activity in the gastrointestinal tract. They also support intestinal barrier function, the thickness of the mucus layer, and intestinal motility. Lactic acid bacteria may therefore help prevent intestinal barrier dysfunction and thus the development of gastrointestinal and systemic disorders.

Therefore, it may be advantageous to provide at least one strain of *Lactiplantibacillus plantarum* and/or *Lacticaseibacillus paracasei* for use in reducing, preventing and/or treating intestinal barrier dysfunction in a subject.

Thus, a twelfth aspect of the disclosure relates to at least one strain of *Lactiplantibacillus plantarum* and/or *Lacticaseibacillus paracasei* for use in reducing, preventing and/or treating intestinal barrier dysfunction in a subject.

By "intestinal barrier dysfunction" we include the meaning of an impaired function of the intestinal lining, which normally acts as a selective barrier to protect the body from harmful substances while allowing the absorption of essential nutrients and fluids. When this barrier is compromised, it can lead to increased intestinal permeability, allowing toxins, pathogens, and undigested food particles to enter the bloodstream. This can trigger an immune response and contribute to various health issues, including chronic inflammation, autoimmune diseases, and gastrointestinal disorders.

A thirteenth aspect of the disclosure relates to the use of at least one strain of *Lactiplantibacillus plantarum* and/or *Lacticaseibacillus paracasei* for reducing, preventing and/or treating intestinal barrier dysfunction in a subject.

A fourteenth aspect of the disclosure relates to the use of at least one strain of *Lactiplantibacillus plantarum* and/or *Lacticaseibacillus paracasei* in the manufacture of a medicament for reducing, preventing and/or treating intestinal barrier dysfunction in a subject.

A fifteenth aspect of the disclosure relates to a method for improving and/or supporting gastrointestinal health in a subject, comprising administering to a subject in need thereof, an effective dose of at least one strain of *Lactiplantibacillus plantarum* and/or *Lacticaseibacillus paracasei.*

By "improving and/or supporting gastrointestinal health" we include the meaning of enhancing the function and balance of the gastrointestinal system by providing at least one strain of *Lactiplantibacillus plantarum* and/or *Lacticaseibacillus paracasei* as a medicine, foods and/or nutritional supplements, which promotes a diverse and balanced gastrointestinal tract microbiome, strengthen the intestinal barrier, and reduce inflammation.

By "gastrointestinal health" we include the meaning of optimal functioning and balance of the gastrointestinal system, including the stomach, intestines, and the gastrointestinal tract microbiome. The term encompasses effective digestion and absorption of nutrients, a strong intestinal barrier, and a balanced microbial community that supports immune function and overall well-being. Good gastrointestinal tract health is essential for preventing digestive issues and reducing inflammation.

A sixteenth aspect of the disclosure relates to at least one strain of *Lactiplantibacillus plantarum* and/or *Lacticaseibacillus paracasei* for improving and/or supporting gastrointestinal health in a subject.

A seventeenth aspect of the disclosure relates to the use of at least one strain of *Lactiplantibacillus plantarum* and/or *Lacticaseibacillus paracasei* for improving and/or supporting gastrointestinal health in a subject.

An eighteenth aspect of the disclosure relates to the use of at least one strain of *Lactiplantibacillus plantarum* and/or *Lacticaseibacillus paracasei* in the manufacture of a medicament for improving and/or supporting gastrointestinal health in a subject.

A nineteenth aspect of the disclosure relates to a method for improving and/or supporting gastrointestinal homeostasis in a subject, comprising administering to a subject in need thereof, an effective dose of at least one strain of *Lactiplantibacillus plantarum* and/or *Lacticaseibacillus paracasei.*

### Gastrointestinal homeostasis

Gastrointestinal homeostasis refers to the balance and stability of the gastrointestinal tract environment, which is crucial for overall health. The gastrointestinal tract is home to a diverse community of microorganisms, collectively known as the gastrointestinal tract microbiota. These microorganisms play vital roles in various bodily functions, including digestion, nutrient absorption, and immune system regulation. Maintaining gastrointestinal homeostasis involves a delicate balance between beneficial and harmful bacteria. When this balance is disrupted, it can lead to dysbiosis, which is associated with various health issues such as inflammatory bowel disease, metabolic syndrome, and obesity.

Probiotics are beneficial bacteria that adhere to the intestinal mucosa, forming a protective barrier against pathogens. This colonization is facilitated by specific surface proteins and polysaccharides that interact with the gastrointestinal tract lining. Probiotics compete with harmful bacteria for nutrients and space, producing antimicrobial substances like bacteriocins and organic acids that inhibit the growth of pathogenic microbes. Additionally, probiotics interact with the host's immune cells, influencing immune responses. They can enhance the production of mucins, which form a protective mucus layer, and produce metabolites like short-chain fatty acids that regulate immune cell activity.

Additionally, probiotics help restore the composition of the gastrointestinal tract microbiota, increasing the abundance of beneficial bacteria and reducing the presence of harmful ones. This restoration is crucial for preventing and managing various inflammatory intestinal diseases. Overall, probiotics has been shown to play a significant role in maintaining gastrointestinal homeostasis by supporting a balanced gastrointestinal tract microbiota, enhancing immune function, and protecting against pathogens.

Therefore, it may be advantageous provide at least one strain of *Lactiplantibacillus plantarum* and/or *Lacticaseibacillus paracasei* for improving and/or supporting gastrointestinal tract homeostasis in a subject.

Thus, a twentieth aspect of the disclosure relates to at least one strain of *Lactiplantibacillus plantarum* and/or *Lacticaseibacillus paracasei* for use in improving and/or supporting gastrointestinal homeostasis in a subject.

By "gastrointestinal homeostasis" we include the meaning of a balanced and stable state of the gastrointestinal system, where all its functions, including digestion, nutrient absorption, and immune response, operate optimally. This balance may be maintained through the regulation of the gastrointestinal tract microbiome, intestinal barrier integrity, and immune system interactions, ensuring overall gastrointestinal tract health and preventing disease.

A twenty-first aspect of the disclosure relates to the use of at least one strain of *Lactiplantibacillus plantarum* and/or *Lacticaseibacillus paracasei* for improving and/or supporting gastrointestinal homeostasis in a subject.

A twenty-second aspect of the disclosure relates to the use of at least one strain of *Lactiplantibacillus plantarum* and/or *Lacticaseibacillus paracasei* in the manufacture of a medicament for improving and/or supporting gastrointestinal homeostasis in a subject.

A twenty-third aspect of the disclosure relates to a method for supporting and/or maintaining gastrointestinal endothelial function in a subject, comprising administering to a subject in need thereof, an effective dose of at least one strain of *Lactiplantibacillus plantarum* and/or *Lacticaseibacillus paracasei.*

By "supporting and/or maintaining gastrointestinal endothelial function" we include ethe meaning of enabling healthy and proper functioning of the endothelial cells lining the blood vessels in the gastrointestinal tract. Healthy endothelial function is crucial for regulating blood flow, reducing inflammation, and maintaining the integrity of the gastrointestinal tract barrier, which helps prevent intestinal barrier dysfunction and chronic inflammation.

By "gastrointestinal endothelial function" we include the meaning of activities of the endothelial cells lining the blood vessels in the gastrointestinal tract. These cells are crucial for regulating blood flow, maintaining vascular tone, and ensuring the integrity of the gastrointestinal tract barrier. Proper gastrointestinal endothelial function supports nutrient and oxygen delivery to the gastrointestinal tract tissues, helps control inflammation, and prevents the entry of harmful substances into the bloodstream.

Endothelial function can be measured using various methods known in the field. These include biomarker analysis, where levels of specific biomarkers such as nitric oxide (NO) and endothelin-1 are measured in blood samples to assess endothelial function. Additionally, advanced imaging techniques like flow-mediated dilation (FMD) and laser Doppler flowmetry can be used to visualize and measure blood flow and vessel dilation.

A twenty-fourth aspect of the disclosure relates to at least one strain of *Lactiplantibacillus plantarum* and/or *Lacticaseibacillus paracasei* for use in supporting and/or maintaining gastrointestinal endothelial function in a subject.

A twenty-fifth aspect of the disclosure relates to the use of at least one strain of *Lactiplantibacillus plantarum* and/or *Lacticaseibacillus paracasei* for supporting and/or maintaining gastrointestinal endothelial function in a subject.

A twenty-sixth aspect of the disclosure relates to the use of at least one strain of *Lactiplantibacillus plantarum* and/or *Lacticaseibacillus paracasei* in the manufacture of a medicament for supporting and/or maintaining gastrointestinal endothelial function in a subject.

### Metabolic health

The disclosure also relates to methods for improving and/or supporting metabolic health in a subject. The disclosure further relates to methods of preventing and/or treating a gastrointestinal condition, a gastrointestinal disorder relating to GI health or to metabolic syndrome and/or a metabolic disease in a subject.

Metabolic health refers to the optimal functioning of the body's metabolic processes, which include the efficient regulation of blood sugar levels, lipid profiles, blood pressure, and insulin sensitivity. Good metabolic health is characterized by balanced energy production and utilization, effective nutrient absorption, and minimal risk of metabolic disorders such as diabetes, obesity, and cardiovascular diseases. Maintaining metabolic health involves a healthy diet, regular physical activity, adequate sleep, and stress management. The common indicator of poor metabolic health is weight gain or obesity where unexpected weight gain or difficulty losing weight can signal metabolic issues. Additionally, high blood sugar levels, elevated blood pressure, imbalances in cholesterol levels, such as high low-density lipoprotein (LDL) and low high-density lipoprotein (HDL), increased abdominal fat, fatigue and low energy level are all symptoms of poor metabolic health.

Therefore, it may be advantageous to provide a method for improving and/or supporting metabolic health in a subject, comprising administering to a subject in need thereof, an effective dose of at least one strain of *Lactiplantibacillus plantarum* and/or *Lacticaseibacillus paracasei.*

A twenty-seventh aspect of the disclosure relates to a method for improving and/or supporting metabolic health in a subject, comprising administering to a subject in need thereof, an effective dose of at least one strain of *Lactiplantibacillus plantarum* and/or *Lacticaseibacillus paracasei.*

By "metabolic health" we include the meaning of the optimal functioning of the body's metabolic processes, which include the efficient regulation of blood sugar levels, blood pressure, cholesterol level, lipid profile, weight, and insulin sensitivity.

A twenty-eighth aspect of the disclosure relates to at least one strain of *Lactiplantibacillus plantarum* and/or *Lacticaseibacillus paracasei* for use in improving and/or supporting metabolic health in a subject.

A twenty-ninth aspect of the disclosure relates to the use of at least one strain of *Lactiplantibacillus plantarum* and/or *Lacticaseibacillus paracasei* for improving and/or supporting metabolic health in a subject.

A thirtieth aspect of the disclosure relates to the use of at least one strain of *Lactiplantibacillus plantarum* and/or *Lacticaseibacillus paracasei* in the manufacture of a medicament for improving and/or supporting metabolic health in a subject.

### Metabolic syndrome

Metabolic syndrome is a condition that greatly raises the risk of developing type II diabetes, heart disease and stroke. The risk factors for developing metabolic syndrome are family history of premature coronary disease, hypertension, hyperlipemia, diabetes and smoking. Age increases the risk of metabolic syndrome, as does male gender and post-menopausal hormonal status. Certain risk factors tend to cluster or occur together and some of these risk factors can be deceased by lifestyle modifications of dietary changes and increased physical activity combined with pharmacologic treatment, which may affect several risk factors simultaneously and thereby reduce the risk of developing metabolic syndrome.

Modulating the gastrointestinal tract microbiota to decrease some of the risk factors of developing metabolic syndromes may be an attractive strategy, as it has been demonstrated in both mice and human studies that there is a close link between changes in gastrointestinal tract microbiota and the development of metabolic syndrome.

Several studies have highlighted the potential of probiotics and prebiotics in treating metabolic syndromes. Probiotics such as Lacticaseibacillus and Bifidobacterium have been shown to inhibit weight gain in obese rats and prevent the rise of non-esterified fatty acids and ketone bodies, likely through their negative regulation of energy metabolism. Experimental studies have demonstrated that Lacticaseibacillus as a supplement is effective in preventing obesity by reducing weight gain and fat accumulation, maintaining normal blood lipid levels, and enhancing the intestinal microbiota by decreasing the ratio of Firmicutes to Bacteroidetes.

In patients with metabolic syndrome, the use of probiotic has been reported to alleviate clinical symptoms and effectively reduce inflammatory biomarkers such as TNF-α. The use of prebiotics and/or probiotics in metabolic syndrome offers numerous benefits, including improvements in metabolic parameters such as BMI, insulin resistance, and inflammatory markers. Additionally, in patients with type II diabetes mellitus, the administration of probiotics has been reported to improve lipid profiles, anthropometric indices, and blood pressure.

Additionally, modulating the gastrointestinal tract microbiota may be an advantageous strategy to reduce risk factors for developing gastrointestinal conditions and disorders. This approach helps balance gastrointestinal tract barrier integrity and mucosal defense, promotes anti-inflammatory responses, and enhances host immune function.

Therefore, it may be advantageous to provide a method preventing and/or treating a gastrointestinal condition, a gastrointestinal disorder relating to GI health or to metabolic syndrome and/or a metabolic disease in a subject, comprising administering to a subject in need thereof, an effective dose of at least one strain of *Lactiplantibacillus plantarum* and/or *Lacticaseibacillus paracasei.*

A thirty-first aspect of the disclosure relates to a method preventing and/or treating a gastrointestinal condition, a gastrointestinal disorder relating to GI health or to metabolic syndrome and/or a metabolic disease in a subject, comprising administering to a subject in need thereof, an effective dose of at least one strain of *Lactiplantibacillus plantarum* and/or *Lacticaseibacillus paracasei.*

By "gastrointestinal condition, a gastrointestinal disorder relating to GI health" we include the meaning of any health issue that affects the gastrointestinal tract, which includes the mouth, esophagus, stomach, intestines, liver, pancreas, and gallbladder. These conditions can range from mild to severe and may involve symptoms such as abdominal pain, flatulence, bloating, constipation, diarrhea and defecation disability. Examples of gastrointestinal disorders include IBS, IBD, ulcerative colitis and microscopic colitis.

By "metabolic syndrome and/or a metabolic disease" we include the meaning of a physical condition that refers to several risk factors specific for cardiovascular disease. Metabolic syndrome covers five metabolic abnormalities, which includes obesity, high blood pressure, high blood triglyceride levels, excess body fat around the waist, low levels of HDL cholesterol, insulin resistance and impaired fasting blood glucose. metabolic disease covers type II diabetes mellitus, obesity, atherosclerosis and cardiovascular disease.

A thirty-second aspect of the disclosure relates to at least one strain of *Lactiplantibacillus plantarum* and/or *Lacticaseibacillus paracasei* for use in preventing and/or treating a gastrointestinal condition, a gastrointestinal disorder relating to GI health or to metabolic syndrome and/or a metabolic disease in a subject.

A thirty-third aspect of the disclosure relates to the use of at least one strain of *Lactiplantibacillus plantarum* and/or *Lacticaseibacillus paracasei* for preventing and/or treating a gastrointestinal condition, a gastrointestinal disorder relating to GI health or to metabolic syndrome and/or a metabolic disease in a subject.

A thirty-fourth aspect of the disclosure relates to the use of at least one strain of *Lactiplantibacillus plantarum* and/or *Lacticaseibacillus paracasei* in the manufacture of a medicament for preventing and/or treating a gastrointestinal condition, a gastrointestinal disorder relating to GI health or to metabolic syndrome and/or a metabolic disease in subject.

A thirty-fifth aspect of the disclosure relates to a method for reducing, preventing and/or treating systemic inflammation in a subject, comprising administering to a subject in need thereof, an effective dose of at least one strain of *Lactiplantibacillus plantarum* and/or *Lacticaseibacillus paracasei.*

By "systemic inflammation" we include the meaning of an inflammatory response that affects the entire body rather than being localized to a specific area. It involves the release of pro-inflammatory cytokines, such as interleukin-1β (IL-1β), interleukin-6 (IL-6), chemokine CXCL-8, interferon-gamma (IFN-γ), tumor necrosis factor (TNF-α) and activation of the immune system, which can lead to symptoms such as fever, increased heart rate, and elevated lymphocyte cell count. Systemic inflammation is associated with various conditions, including autoimmune diseases, Crohn's disease and ulcerative colitis, cardiovascular disease and diabetes.

Systemic inflammation is measured by and elevated C-Reactive Protein (CRP) in the blood, which is a protein produced by the liver that is increases when inflammation is present. An elevated CRP level is above 3 mg/L and a normal CRP level is less than 3 mg/L.

A thirty-sixth aspect of the disclosure relates to at least one strain of *Lactiplantibacillus plantarum* and/or *Lacticaseibacillus paracasei* for use in reducing, preventing and/or treating systemic inflammation in a subject.

A thirty-seventh aspect of the disclosure relates to the use of at least one strain of *Lactiplantibacillus plantarum* and/or *Lacticaseibacillus paracasei* for reducing, preventing and/or treating systemic inflammation in a subject.

A thirty-eighth aspect of the disclosure relates to the use of at least one strain of *Lactiplantibacillus plantarum* and/or *Lacticaseibacillus paracasei* in the manufacture of a medicament reducing, preventing and/or treating systemic inflammation in a subject.

### Pro-inflammatory cytokines

Pro-inflammatory cytokines are signaling proteins essential for the body's immune response, as they promote inflammation. These cytokines are produced by various cells, including immune cells like macrophages and lymphocytes, as well as non-immune cells such as endothelial cells. Key pro-inflammatory cytokines include IL-1β, IL-6, CXCL-8, IFN-γ, and TNF-α. They are released from immune cells to initiate an immune response, attracting additional immune cells to the site of inflammation to help remove pathogens and damaged cells. These cytokines also amplify the inflammatory response by promoting the production of more cytokines and other inflammatory mediators, ensuring a robust immune reaction.

Elevated expression of pro-inflammatory cytokines in the gastrointestinal tract is often associated with various inflammatory conditions, such as IBD and infections. In the gastrointestinal tract, pro-inflammatory cytokines can increase vascular permeability, allowing immune cells to migrate to the site of inflammation. However, long-term elevated cytokine levels can disrupt gastrointestinal tract barrier function, leading to increased permeability and further inflammation. This persistent inflammation can contribute to the symptoms and progression of inflammatory gastrointestinal tract diseases.

The inventors of the present invention have surprisingly found that culturing probiotic strain *Lactiplantibacillus plantarum* with pro-inflammatory cytokines results in enhanced growth of *Lactiplantibacillus plantarum,* as shown in Example 1. This increased growth may provide an anti-inflammatory effect in the gastrointestinal tract, protecting the gastrointestinal tract barrier function and alleviating symptoms related to persistent inflammation in the gastrointestinal tract.

Therefore, it may be advantageous to provide a method for increasing growth of at least one strain of *Lactiplantibacillus plantarum* in the gastrointestinal tract of a subject having an elevated expression of one or more pro-inflammatory cytokines, comprising administering to a subject in need thereof, an effective dose of at least one strain of *Lactiplantibacillus plantarum.*

A thirty-ninth aspect of the disclosure relates to a method for increasing growth of at least one strain of *Lactiplantibacillus plantarum* in the gastrointestinal tract a subject having an elevated expression of one or more pro-inflammatory cytokines, comprising administering to a subject in need thereof, an effective dose of at least one strain of *Lactiplantibacillus plantarum.*

By "elevated expression of one or more pro-inflammatory cytokines" we include the meaning of elevated levels of a pro-inflammatory cytokine selected from the group consisting of IL-1β, IL-6, CXCL-8, IFN-γ, TNF-α and any combination thereof. Specifically, elevated expression is defined as IL-1β levels >5 ng/L, IL-6 levels >8 ng/L, CXCL-8 levels >80 ng/L, IFN-γ levels >8.6 pg/ml and TNF-α levels >15 ng/L. Elevated expression of pro-inflammatory cytokines can be measured by several methods including Enzyme-Linked Immunosorbent Assay (ELISA), ELIspot, intracellular flow cytometry and multiplex immunoassays and in a body fluid selected from the group consisting of blood, saliva, faces, intestinal tissue and any combination thereof. Preferably, the elevated expression of pro-inflammatory cytokines is measured using ELISA.

A fortieth aspect of the disclosure relates to at least one strain of *Lactiplantibacillus plantarum* for use in increasing growth of at least one strain of *Lactiplantibacillus plantarum* in the gastrointestinal tract of a subject having an elevated expression of one or more pro-inflammatory cytokines.

A forty-first aspect of the disclosure relates to the use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A forty-second aspect of the disclosure relates to a non-therapeutic use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A forty-third aspect of the disclosure relates to the use of at least one strain of *Lactiplantibacillus plantarum* in the manufacture of a medicament for increasing growth of at least one strain of *Lactiplantibacillus plantarum* in the gastrointestinal tract of a subject having an elevated expression of one or more pro-inflammatory cytokines.

A forty-fourth aspect of the disclosure relates to the use of at least one strain of *Lactiplantibacillus plantarum* in the manufacture of a supplement for increasing growth of at least one strain of *Lactiplantibacillus plantarum* in the gastrointestinal tract of a subject having an elevated expression of one or more pro-inflammatory cytokines.

The inventors of the present invention have surprisingly found that culturing probiotic strains of *Lactiplantibacillus plantarum* and *Lacticaseibacillus paracasei* with pro-inflammatory cytokines results in increased biofilm formation, as shown in Example 1. This biofilm may provide an anti-inflammatory effect in the gastrointestinal tract, protecting the gastrointestinal tract barrier function and alleviating symptoms related to persistent inflammation in the gastrointestinal tract.

The inventors of the present invention have surprisingly found that culturing *Lactiplantibacillus plantarum* with pro-inflammatory cytokines results in increased biofilm formation, as shown in Example 1, increased growth of *Lactiplantibacillus plantarum* and in particular growth of *Lactiplantibacillus plantarum* DSM 9843 and *Lactiplantibacillus plantarum* DSM 15312. Increased growth may be measured *in vitro* by a growth assay, as described in Example 1, where bacteria are incubated in a microtiter plate for 24 hours in the presence of cytokines. The growth may be quantified by optic density at 600nm using microplate reader (Cytation 3, Biotek Inc).

Therefore, it may be advantageous to provide a method for increasing the biofilm formation in the gastrointestinal tract, wherein the biofilm comprises *Lactiplantibacillus plantarum* and/or *Lacticaseibacillus paracasei.*

Therefore, in one embodiment the present invention relates to the method, the at least one strain or the use as described herein, wherein the biofilm comprises *Lactiplantibacillus plantarum* and/or *Lacticaseibacillus paracasei.* Preferably, the biofilm is formed in gastrointestinal tract after at least two weeks of administration of the at least one strain of *Lactiplantibacillus plantarum* and/or *Lacticaseibacillus paracasei* to the subject.

Another embodiment of the present invention relates to the method, the at least one strain or the use as described herein, wherein the subject has an elevated level of pro-inflammatory cytokines in a body fluid selected from the group consisting of blood, saliva, feces, intestinal tissue and combinations thereof.

Elevated levels of pro-inflammatory cytokines can be measured in body fluids and tissues. The threshold levels of these cytokines vary depending on the type of body fluid, such as blood, saliva, and feces, as well as the specific tissue, such as intestinal tissue. As mentioned earlier elevated levels of pro-inflammatory cytokines are defined herein as IL-1β >5 ng/L, IL-6 >8 ng/L, CXCL-8 >80 ng/L, IFN-γ >8.6 pg/ml, and TNF-α >15 ng/L. Additionally, a CRP level of >3 mg/L is required in combination with the elevated levels of pro-inflammatory cytokines to define an inflammatory state in a subject. Pro-inflammatory cytokines in blood, saliva, faeces and intestinal tissue can be measured using ELISA or multiplex immunoassays.

A further embodiment of the present invention relates to the method, the at least one strain or the use as described herein, wherein the subject has an elevated expression of one or more pro-inflammatory cytokine(s) in intestinal cells or in the intestine.

In particular, increased growth was observed when *Lactiplantibacillus plantarum* strains were cultured in the presence of pro-inflammatory cytokines IFN-γ, IL-6, IL-1β, and CXCL8. These cytokines induced significant growth increases in the *Lactiplantibacillus plantarum* strains, *Lactiplantibacillus plantarum* DSM 9843 and *Lactiplantibacillus plantarum* DSM 15312, as shown in Example 1. Additionally, increased biofilm formation was observed for both *Lactiplantibacillus plantarum* and *Lacticaseibacillus paracasei* strains in Example 1. The capacity of *Lactiplantibacillus plantarum* DSM 9843 to form biofilm was significantly increased when cultured in the presence of IL-1β, TNF-α, IFN-γ, CXCL8 and IL-6. In particular, IL-1β had the largest effect on the biofilm formation of *Lactiplantibacillus plantarum* DSM 9843. Furthermore, *Lactiplantibacillus plantarum* DSM 15312 showed increased biofilm formation when cultured in the presence of IL-1β, and *Lacticaseibacillus paracasei* DSM 13434 demonstrated increased biofilm formation when cultured in the presence of CXCL8.

Therefore, it may be advantageous to provide a method as described herein, wherein one or more pro-inflammatory cytokines is selected from the group consisting of IL-1β, IL-6, CXCL-8, IFN-γ, TNF-α and any combination thereof.

Thus, a further embodiment of the present invention relates to the method, the at least one strain or the use as described herein, wherein the one or more pro-inflammatory cytokines are selected from the group consisting of interleukin-1β (IL-1β), interleukin-6 (IL-6), chemokine (CXCL-8), interferon-gamma (IFN-γ), tumor necrosis factor (TNF-α) and any combination thereof.

IL-1β is a key pro-inflammatory cytokine that plays a crucial role in the body's immune response and inflammation. Produced primarily by activated macrophages, monocytes, and dendritic cells, IL-1β is synthesized as an inactive precursor and requires cleavage by caspase-1 within inflammasomes to become active. IL-1β is one of the first cytokines released during an inflammatory response, helping to initiate and amplify inflammation by promoting the production of other cytokines and inflammatory mediators. It influences the activity of various immune cells, including T cells, and can promote the differentiation of T helper cells into the pro-inflammatory Th17 subset, which is involved in autoimmune inflammation. Elevated levels of IL-1β can disrupt the gastrointestinal tract barrier function by damage the gastrointestinal tract lining, leading to increased permeability and further inflammation, a disruption associated with various gastrointestinal tract-related diseases such as IBD.

A further embodiment of the present invention relates to the method, the at least one strain or the use as described herein, wherein the pro-inflammatory cytokine is interleukin-1β is (IL-1β).

A further embodiment of the present invention relates to the method, the at least one strain or the use as described herein, wherein the pro-inflammatory cytokine is TNF-α. A further embodiment of the present invention relates to the method, the at least one strain or the use as described herein, wherein the pro-inflammatory cytokine is IFN-γ. A further embodiment of the present invention relates to the method, the at least one strain or the use as described herein, wherein the pro-inflammatory cytokine is CXCL8. A further embodiment of the present invention relates to the method, the at least one strain or the use as described herein, wherein the pro-inflammatory cytokine is IL-6.

Another embodiment of the present invention relates to the method, the at least one strain or the use as described herein, wherein the subject is a mammal, preferably wherein the subject is human.

Yet, another embodiment of the present invention relates to the method, the at least one strain or the use as described herein, wherein the disorder is a gastrointestinal disorder and wherein the gastrointestinal disorder is selected from the group consisting of irritable bowel syndrome (IBS), inflammatory bowel disease (IBD), or any of the symptoms often included in these disorders; abdominal pain, flatulence, bloating, constipation, diarrhea and defecation disability.

By "irritable bowel syndrome (IBS)" we include the meaning of a condition that affects the gastrointestinal tract, including the stomach and intestines. It is characterized by a group of symptoms such as cramping, abdominal pain, bloating, gas, and changes in bowel habits, including diarrhea and constipation. IBS is a chronic condition that requires long-term management. It can be diagnosed through a combination of tests and physical examinations. Blood tests are used to identify infections and anemia, and stool tests are used to detect infections and blood. The diagnostic criteria for IBS include abdominal pain and discomfort occurring at least one day a week over the past three months, pain related to defecation, and changes in stool frequency and consistency. Additionally, endoscopic procedures such as colonoscopy and upper endoscopy may be used to examine the gastrointestinal tract.

By "inflammatory bowel disease (IBD)" we include the meaning of a group of chronic conditions that cause chronic inflammation and tissue damage in the gastrointestinal tract, such as Crohn's disease, ulcerative colitis and microscopic colitis. Symptoms of IBD include abdominal pain, flatulence, bloating, constipation, diarrhea, and difficulty with defecation. IBD can be diagnosed through a combination of tests, including blood test, stool tests and tissue biopsies to detect pro-inflammatory markers, such as elevated levels of IL-1β, IL-6, CXCL-8, IFN-γ, and TNF-α. Stool tests can also identify infection, blood and mucus in the stool. Additionally, endoscopic procedures like colonoscopy and upper endoscopy are used to examine the gastrointestinal tract and collect tissue biopsies for detecting inflammatory markers.

A further embodiment of the present disclosure relates to the method, the at least one strain or the use as described herein, wherein inflammatory bowel disease (IBD) is selected from Chron's disease, ulcerative colitis, microscopic colitis and any combination thereof.

### Gastrointestinal dysbiosis

Gastrointestinal dysbiosis refers to an imbalance in the microbial community within the gastrointestinal tract. This condition occurs when there is a disruption in the diversity and abundance of gastrointestinal tract microorganisms, including bacteria, viruses, and fungi. Dysbiosis can lead to various gastrointestinal symptoms such as bloating, gas, diarrhea, and constipation. It is also associated with more severe health issues, including IBD, IBS, and metabolic disorders. Factors contributing to gastrointestinal tract dysbiosis include poor diet, antibiotic use, stress, and infections. Restoring a healthy gastrointestinal tract microbiome through dietary changes, probiotics, and prebiotics can help alleviate symptoms and improve overall health.

Yet, a further embodiment of the present disclosure relates to the method, the at least one strain or the use as described herein, wherein the condition is a dysbiosis and wherein the dysbiosis is a gastrointestinal dysbiosis.

By "gastrointestinal dysbiosis" we include the meaning of an imbalance in the microbial community of the gastrointestinal tract, often leading to digestive issues and associated with various health conditions such IBD, IBS, and metabolic disorders. Gastrointestinal tract dysbiosis can be measured using various methods that analyse the composition and function of the gastrointestinal tract microbiota. These methods include stool tests, which analyse the microbial content of stool samples, and dysbiosis indexes that quantify the imbalance in gastrointestinal tract microbial communities. These indexes often involve large-scale bacterial marker profiling, taxon-based methods, and diversity measures. Biomarkers in body fluids and tissues can also indicate dysbiosis. For example, elevated levels of calprotectin in stool samples can indicate inflammation and gastrointestinal tract microbiota imbalance. Further, clinical symptoms such as bloating, diarrhea, constipation, and abdominal pain can help diagnose gastrointestinal tract dysbiosis. These symptoms, combined with microbiome analysis, provide a comprehensive overview of gastrointestinal tract health.

Thus, a further embodiment of the present disclosure relates to the method, the at least one strain or the use as described herein, wherein the gastrointestinal dysbiosis is a microbial dysbiosis in the gastrointestinal tract.

By "microbial dysbiosis" we include the meaning of an imbalance in the microbial communities within a specific environment, such as the human gastrointestinal tract, skin, or other body sites. This imbalance can result from a disruption in the diversity and composition of microorganisms, leading to a predominance of harmful microbes and a reduction in beneficial microbes. In the human gastrointestinal tract, microbial dysbiosis can affect digestion, immune function, and overall health. It is associated with various conditions, including IBD, IBS, and metabolic disorders.

Another embodiment of the present disclosure relates to the method, the at least one strain or the use as described herein, wherein the systemic inflammation is associated with one or more disease(s), one or more disorder(s), one or more syndrome(s) and/or one or more condition(s).

By the term "disease" we include the meaning of a specific medical condition that impairs the normal functioning of the body. A disease is characterized by specific signs and symptoms and can be caused by various factors, including infections, genetic mutations, environmental influences, and lifestyle choices. Diseases can affect any part of the body and may be acute (short-term) or chronic (long-term). A disease may be inflammatory IBD and metabolic disease.

By the term "disorder" we include the meaning of a disruption of normal physical or mental functions, often characterized by a set of symptoms and signs. A disorder may not have an identifiable cause or origin and can result from a combination of genetic, environmental, and lifestyle factors. A disorder may be a gastrointestinal disorder.

By the term "syndrome" we include the meaning a collection of symptoms and signs that occur together and characterize a particular abnormality or condition. Syndromes may not have a single identifiable origin and can result from a variety of underlying factors. Syndromes can affect multiple systems in the body and may be associated with genetic and environmental causes. A syndrome may be metabolic syndrome.

By the term "condition" we include the meaning of a state of health that deviates from the normal functioning of the body. Conditions can be temporary or chronic and may result from various causes, including genetic factors, infections, environmental influences, or lifestyle choices. Conditions may encompass a range of health issues.

Yet, another embodiment of the present disclosure relates to the method, the at least one strain or the use as described herein, wherein the disease is a metabolic disease and wherein the metabolic disease is selected from the group consisting of type 2 diabetes mellitus, obesity, atherosclerosis and cardiovascular disease.

By "type 2 diabetes mellitus" we include the meaning of a chronic condition characterized by high levels of glucose in the blood. It occurs when the body becomes resistant to insulin or when the pancreas does not produce enough insulin. As a result, glucose builds up in the blood instead of being used for energy. Type 2 diabetes mellitus can be diagnosed using several tests that measures the blood glucoses level, such as haemoglobin A1c test, which measures the average blood glucose levels over the past two to three months. The haemoglobin A1c level may be determined by high performance liquid chromatography and the inulin level may be determined by an ELISA.

A normal haemoglobin A1c level in the blood plasma is considered to be in the range of 4% to 5.6%. A haemoglobin A1c level within the range of 5.7% to 6.4% is considered to be prediabetic. Type 2 diabetes mellitus is diagnosed if the haemoglobin A1c level is of 6.5% or above.

By "obesity" we include the meaning of a medical condition characterized by excessive body fat accumulation that may negatively impact health. It is typically defined by a Body Mass Index (BMI) of 30 or higher. Obesity increases the risk of various health issues, including heart disease, diabetes, hypertension, and certain cancers. It results from a combination of genetic, behavioural, and environmental factors, including poor diet, lack of physical activity, and metabolic disorders. Obesity is diagnosed using BMI. A BMI in the range of 30 to 39.9 indicates obesity and a normal weight is defined by a BMI in the range of 18.5 to 24.9.

The obesity is preferably abdominal obesity, which includes having a waist circumference of more than 35 inches for women and more than 40 inches for men. An increased waist circumference is the form of obesity most strongly tied to metabolic syndrome.

By "atherosclerosis" we include the meaning of a condition where plaque builds up inside the arteries. This plaque is made up of fats, cholesterol, calcium, and other substances found in the blood. Over time, the plaque hardens and narrows the arteries, reducing the blood flow to various organs and tissue. The narrowing of the arteries can lead to serious health issues, such as heart attacks, strokes, and other cardiovascular diseases.

Atherosclerosis is diagnosed using blood tests and heart imaging tests. Imbalances in cholesterol levels such as increased total cholesterol, increased triglycerides, increased low-density lipoprotein (LDL) cholesterol, decreased high-density lipoprotein (HDL) cholesterol, and increased blood sugar may indicate atherosclerosis. Blood sugar levels can be measured by the haemoglobin A1c test, as previously described. Cholesterol, triglyceride, and lipoprotein levels can be measured by blood sampling of peripheral blood and analysed by nuclear magnetic resonance spectroscopy. Additionally, various heart imaging tests can be used to visualise the arteries and detect plaque buildup. These imaging tests include angiography, cardiac magnetic resonance imaging (MRI), and cardiac positron emission tomography (PET) scanning.

By "cardiovascular disease" we include the meaning of a group of disorders affecting the heart and blood vessels. These include coronary heart disease, cerebrovascular disease, peripheral arterial disease, rheumatic heart disease, congenital heart disease, deep vein thrombosis, and pulmonary embolism.

Cardiovascular diseases are often caused by a blockage that prevents blood from flowing to the heart, typically due atherosclerosis and an increased risk of blood clots. It may also be associated with damage to arteries in organs such as the brain, heart, kidney and eyes. Common risk factors include unhealthy diet, physical inactivity, tobacco use, and harmful use of alcohol.

Taken together, metabolic disease such as type 2 diabetes mellitus, obesity, atherosclerosis and cardiovascular disease are some of the main causes of deaths and disabilities globally and can often be prevented by committing to a heathier lifestyle. However, since incidences of metabolic diseases are increasing each year, there is a need for new or alternative method for preventing and/or treating metabolic disease.

A further embodiment of the present disclosure relates to the method, the at least one strain or the use as described herein, wherein the syndrome is metabolic syndrome and wherein the metabolic syndrome is selected from the group consisting of increased blood pressure, high blood sugar level, excess body fat around the waist and abnormal cholesterol or triglyceride levels.

By "increased blood pressure" we include the meaning of a blood pressure that is above the normal range. The term increased blood pressure is also to be understood as hypertension, which occurs when the force of blood flowing through the blood vessels is consistently too high. This condition can lead to serious health issues such as heart disease and stroke. Blood pressure is measured in millimetres of mercury, within the major arterial system of the body. It is separated into systolic and diastolic pressure, where systolic pressure is the maximum blood pressure during contraction of the ventricle and diastolic pressure is the minimum blood pressure measured just prior to the next contraction. The blood pressure is written as the systolic pressure over the diastolic pressure, e.g. 120/80 mm Hg (millimetres of mercury). Blood pressure may be measured by automatic electronic sphygmomanometer after resting for more than 5 minutes. Increased blood pressure is considered to be a blood pressure of 130/80 mm Hg or higher and a normal blood pressure is considered to be a blood pressure in the range of 90/60 mm Hg to 120/80 mm Hg.

By "high blood sugar level" we include the meaning of hyperglycemia, which occurs when there is too much glucose in the blood. This condition is commonly associated with diabetes. Hyperglycemia is defined as a fasting blood glucose level greater than 125 mg/dL (milligrams per deciliter) after not eating for at least eight hours. A normal blood sugar level is defined a fasting blood glucose level less or equivale to 100 mg/dl. The blood sugar level in the blood plasma may be measured by enzymatic methods, such as hexokinase, glucose oxidase and glucose dehydrogenase

By "excess body fat around the waist" we include the meaning of having a waist circumference of more than 35 inches for women and more than 40 inches for men. An increased waist circumference is the form of obesity most strongly tied to metabolic syndrome.

By "abnormal cholesterol" we include the meaning of having levels of lipid in the blood that are either too high or too low. Cholesterol is a type of lipid or fat in the blood. An increased total cholesterol level is considered to be 200 mg/dL or higher, while a normal total cholesterol level is less than 200 mg/dL. An increased LDL cholesterol level is considered to be 100 mg/dL or higher, while a normal LDL level is below 100 mg/dL. A decreased HDL cholesterol level is considered to be below 40 mg/dL, while a normal HDL level is 60 mg/dL or higher. cholesterol levels may be measured by blood sampling of peripheral blood and analysed by nuclear magnetic resonance spectroscopy.

By "abnormal triglyceride levels" we include the meaning of high or increased triglyceride levels of more than 150 mg/dL. Triglyceride is a type of lipid or fat in the blood. Increased triglyceride level is considered to be in the range of 150-500 mg/dL and normal triglyceride level is considers to be below 150 mg/dL. Triglyceride levels may be measured by blood sampling of peripheral blood and analysed by nuclear magnetic resonance spectroscopy.

Another embodiment of the present disclosure relates to the method, the at least one strain or the use as described herein, wherein the at least one strain of *Lactiplantibacillus plantarum* can adhere to the intestinal epithelium and persist in the intestine.

Preferably, the at least one strain of *Lactiplantibacillus plantarum* is able to adhere to the intestinal epithelium and persist in the intestine. Particular strains of this species comprise a mannose-specific adhesin (Adlerberth et al, 1996, Appl Environ Microbiol, 62(7):2244-2251), including *Lactiplantibacillus plantarum* selected from the group consisting of *Lactiplantibacillus plantarum* DSM 6595 (299), *Lactiplantibacillus plantarum* DSM 9843 (299v), *Lactiplantibacillus plantarum* DSM 15312 (HEAL 9), *Lactiplantibacillus plantarum* DSM 15313 (HEAL 19), *Lactiplantibacillus plantarum* DSM 15316 (HEAL 99) and any combination thereof.

Yet, another embodiment of the present disclosure relates to the method, the at least one strain or the use as described herein, wherein the at least one strain of *Lactiplantibacillus plantarum* comprises a mannose-specific adhesin.

The at least one *Lactiplantibacillus* and/or *Lacticaseibacillus*/*Lactobacillus* strain according to the present disclosure may be viable, inactivated or dead. Preferably, the strain is viable. For example, preferably the strain is freeze-dried.

Advantageously, the at least one strain of *Lactiplantibacillus* and/or *Lacticaseibacillus* is a probiotic strain.

Probiotic bacteria are defined as "live microorganisms that, when administered in adequate amounts, confer a health benefit on the host" (Hill et al, Nat Rev Gastroenterol Hepatol, 2014, 11(8):506-514). For a bacterium to fulfil the definition of a probiotic it typically has to be able to survive in and colonise the intestines, survive the processes of production and storage, and have evidence that it has positive effects on consumer health.

By "probiotic strain" we include the meaning of a strain of bacteria which when administered in adequate amounts confer a health benefit on the host. Typically, the administration of said at least one probiotic strain will alter the composition of the gastrointestinal tract microbiota.

By "at least one strain" we include the meaning of one or more strain(s) of bacteria which when administered in adequate amounts confer a health benefit on the host. Preferably the "at least one strain" according to the present disclosure is at least one strain of *Lactiplantibacillus plantarum* DSM 9843 and/or *Lacticaseibacillus paracasei* DSM 13434.

The *Lacticaseibacillus paracasei* species was formerly known by the name *Lactobacillus paracasei.* The terms *"Lacticaseibacillus paracasei", "L. paracasei"* and *"Lactobacillus paracasei"* are used interchangeably and refers to the same.

The *Lactiplantibacillus plantarum* species was formerly known by the name *Lactobacillus plantarum.* The terms *"Lactiplantibacillus plantarum", "L. plantarum"* and *"Lactobacillus plantarum"* are used interchangeably and refers to the same.

Yet, another embodiment of the present disclosure relates to the method, the at least one strain or the use as described herein, wherein the at least one strain of *Lactiplantibacillus plantarum* is selected from *Lactiplantibacillus plantarum* DSM 6595 (299^{™}), *Lactiplantibacillus plantarum* DSM 9843 (299v^{™}), *Lactiplantibacillus plantarum* DSM 15312 (HEAL 9^{™}), *Lactiplantibacillus plantarum* DSM 15313 (HEAL 19^{™}), *Lactiplantibacillus plantarum* DSM 15316 (HEAL 99^{™}), *Lactiplantibacillus plantarum* DSM 32131 (GOS42^{™}), *Lactiplantibacillus plantarum* DSM 17852 (LB3e^{™}) and *Lactiplantibacillus plantarum* DSM 17853 (LB7c^{™}).

In another embodiment the at least one strain of *Lactiplantibacillus plantarum* is selected from the group consisting of *Lactiplantibacillus plantarum* DSM 6595 (299^{™}), *Lactiplantibacillus plantarum* DSM 9843 (299v^{®}), *Lactiplantibacillus plantarum* DSM 15312 (HEAL 9^{™}) and any combination thereof.

In a preferred embodiment the least one strain of *Lactiplantibacillus plantarum* is selected from *Lactiplantibacillus plantarum* DSM 9843 (299v^{®}), *Lactiplantibacillus plantarum* DSM 15312 (HEAL 9^{™}) and any combination thereof.

In another embodiment the at least one strain of *Lactiplantibacillus plantarum* is *Lactiplantibacillus plantarum* DSM 9843 (299v^{®}).

In another embodiment the at least one strain of *Lactiplantibacillus plantarum* is *Lactiplantibacillus plantarum* DSM 15312 (HEAL 9^{™}).

Yet, another embodiment of the present disclosure relates to the method, the at least one strain or the use as described herein, wherein the at least one strain of *Lacticaseibacillus paracasei* is selected from *Lacticaseibacillus paracasei* DSM 13434 (8700:2^{™}) or *Lacticaseibacillus paracasei* DSM 13432 (02A^{™}). Preferably, wherein the at least one strain of *Lacticaseibacillus paracasei* is *Lacticaseibacillus paracasei* DSM 13434 (8700:2^{™}).

In another embodiment he at least one strain of *Lacticaseibacillus paracasei* is *Lacticaseibacillus paracasei* DSM 13434 (8700:2^{™}).

*Lactobacillus paracasei* DSM 13434 (8700:2^{™}) and *Lactobacillus paracasei* DSM 13432 (02:A^{™}) were deposited on 6 April 2000 at DSMZ-Deutsche Sammlung von Mikroorganismen und Zellkulturen GmbH, Mascheroder Weg 1b, D-38124 Braunschweig, Germany, by Probi AB.

*Lactobacillus plantarum* DSM 6595 (299^{™}) was deposited on 2 July 1991 at DSM-Deutsche Sammlung von Mikroorganismen und Zellkulturen GmbH, Mascheroder Weg 1 B, D-3300 Braunschweig, Germany, in the name of Probi (i.e. Probi AB).

*Lactobacillus plantarum* DSM 9843 (299V^{®}) was deposited on 16 March 1995 at DSM-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* DSM 15312 (HEAL 9^{™}), *Lactobacillus plantarum* DSM 15313 (HEAL 19^{™}), and *Lactobacillus plantarum* DSM 15316 (HEAL 99^{™}), were deposited on 27 November 2002 at DSMZ-DEUTSCHE SAMMLUNG VON MIKROORGANISMEN UND ZELLKULTUREN GmbH, Mascheroder Weg 1b, D-38124 Braunschweig, Germany, by Probi AB.

*Lactobacillus plantarum* DSM 32131 (GOS42^{™}) was deposited on 2 September 2015 at Leibniz Institute DSMZ-German Collection of Microorganisms and Cell Cultures, Inhoffenstr. 7 B, D-38124 Braunschweig, Germany by Probi AB.

*Lactobacillus plantarum* DSM 17852 (LB3e^{™}) and *Lactobacillus plantarum* DSM 17853 (LB7c^{™}) were deposited on 6 January 2006 at DSMZ-Deutsche Sammlung von Mikroorganismen und Zellkulturen GmbH, Mascheroder Weg 1b, D-38124 Braunschweig, Germany, by Probac AB. All rights and duties in connection with microorganism deposits DSM 17852 and DSM 17853 were subsequently given to and accepted by Probi AB, who is now the depositor of the DSM 17852 and DSM 17853 strains.

The subject may be instructed to consume a therapeutically effective amount of the at least one probiotic strain according to the method, the at least one strain or the use as described herein in combination with water, another aqueous solvent or a food product.

Another embodiment of the present disclosure relates to the method, the at least one strain or the use as described herein, wherein the at least one strain is administered at a daily dose of from about 1 x 10⁶ to about 1 x 10¹⁴ colony forming units (CFU) and/or about 1 x 10⁹ to about 1 x 10¹¹ CFU. In a preferred embodiment of the present disclosure, the daily dose of the at least one strain is 1 x 10¹⁰ CFU.

It will be appreciated that a preferable daily dose may also be achieved by administration of more than one sub-dose, for example, by a twice daily administration of a unit dose comprising half of the preferable daily dose. Hence, the preferred ranges for the effective dose may also represent the preferred daily dosage to be achieved in whatever number of unit doses is practical.

If the at least one strain consists of more than one strain, such amounts represent the total CFU/dose of the combination of strains.

A further embodiment of the present disclosure relates to the method, the at least one strain or the use as described herein, wherein the at least one strain is administered daily and/or in a single unit dosage form.

The composition according to the disclosure may be a dietary supplement. By "dietary supplement" we include the meaning of a manufactured product intended to supplement the diet when taken by mouth, e.g. as a pill, capsule, tablet, or liquid. Dietary supplements may contain substances that are essential to life and/or those that have not been confirmed as being essential to life but may have a beneficial biological effect. When the composition according to the disclosure is in the form of a dietary supplement the carrier(s) to be added include those well known to a skilled person in the art, for example those given in Remington: The Science and Practice of Pharmacy, 19th ed., vol. 1 & 2 (ed. Gennaro, 1995, Mack Publishing Company*).* Any other ingredients that are normally used in dietary supplements are known to a skilled person and may also be added conventionally together with the strain.

Yet, a further embodiment of the present disclosure relates to the method, the at least one strain or the use as described herein, wherein the at least one strain is administered daily for at least one week, such as for at least two weeks, e.g. for at least three weeks, such as at least four weeks, e.g. for at least five weeks, such as at least six weeks, e.g. at least seven weeks, at least eight weeks, e.g. at least nine weeks, at least ten weeks, such as at least eleven weeks or at least twelve weeks. In a preferred embodiment of the present disclosure, the at least one strain or the use as described herein, wherein the at least one strain is administered daily for at least two weeks.

Another embodiment of the present disclosure relates to the method, the at least one strain or the use as described herein, wherein the at least one strain of *Lactiplantibacillus plantarum* and/or *Lacticaseibacillus paracasei* is alive, dead or attenuated (e.g. a postbiotics and/or paraprobiotic and/or heat-inactivated), preferably wherein the at least one stain is alive.

By "alive" we include the meaning of a probiotic bacterial strain that is viable when cultured on e.g. agar plates and wherein its viability is measured using plate counts.

By "dead" we include the meaning of a probiotic bacterial strain that is not viable when cultured on e.g. agar plates and wherein its non-viability is measured using plate counts.

By "attenuated" we include the meaning of a probiotic bacterial strain that has been weakened or modified to reduce its viability. This process of attenuation ensures that the probiotic can still confer health benefits without posing any risk to the host.

Yet, another embodiment of the present disclosure relates to the method, the at least one strain or the use as described herein, wherein the at least one strain is administered in the form of a composition comprising at least one pharmaceutically and/or nutritionally acceptable carrier, excipient and/or diluent material.

The at least one strain of *Lactiplantibacillus plantarum* and/or *Lacticaseibacillus paracasei* according to any aspect of the disclosure may be present in a composition comprising at least one pharmaceutically and/or nutritionally acceptable carrier, diluent or excipient material. The composition may be as a solid or liquid formulation, and hence the at least one carrier may be a solid or a liquid, or may comprise both at least one solid component and at least one liquid component.

Examples of a suitable liquid carrier include water, milk, coconut water, fruit drinks and juices, milk substitutes (soya drink, oat drink, nut and other plant-based drinks), sparkling beverages, oil formulations including one or more of a nut or vegetable oil, such as coconut, rapeseed, olive, palm, corn/maize; glycerin, propylene glycol; and aqueous solvents.

Examples of a suitable solid carrier or excipient include maltodextrin, inulin, a cellulose such as microcrystalline cellulose (MCC), hydroxypropylmethylcellulose (HPMC) or hydroxy-propylcellulose (HPC), sugar alcohols, high molecular weight polyethylene glycols, lactose, sodium citrate, calcium carbonate, dibasic calcium phosphate and glycine, disintegrants such as starch (preferably corn, potato, tapioca or other vegetable starch), sodium starch glycollate, croscarmellose sodium and certain complex silicates, and granulation binders such as polyvinylpyrrolidone, sucrose, gelatin and acacia. Additionally, lubricating agents such as magnesium stearate, stearic acid, glyceryl behenate and talc may be included.

The carrier may be selected from a pharmaceutically acceptable carrier, excipient, or diluent; and a nutritionally acceptable i.e. food-grade carrier, excipient, or diluent material. For example, the carrier material may be a food.

Examples of suitable "pharmaceutically acceptable" carriers, excipients and diluents include those well known to a skilled person in the art, for example those given in Remington: The Science and Practice of Pharmacy, 19th ed., vol. 1 & 2 (ed. Gennaro, 1995, Mack Publishing Company*).*

By "nutritionally acceptable" or "food-grade" we include carriers, ingredients and excipients that meet the 'generally recognized as safe' (GRAS) criteria.

By "food" we include any substance for consumption to provide nutritional benefit or support for an organism. Examples of suitable food carriers include beverages (e.g. juices), dairy products (e.g. yoghurts, cheese, ice creams, infant formula and spreads such as margarine), dairy-alternative products (e.g. soy, nut or other plant-based drinks, yoghurts and spreads), cereal-based products (e.g. breads, biscuits, breakfast cereals, pasta and dry food bars such as health bars), and baby food (e.g. pureed fruit and/or vegetable).

The composition according to the disclosure may be a dry, non-fermented composition, a fermented composition, or a dry, fermented composition. Fermentation in this context particularly includes lactic acid fermentation by lactic acid bacteria in anaerobic conditions. In the case of a dry, non-fermented composition, substantially no fermentation takes place before ingestion by a subject, and so fermentation only takes place in the gastrointestinal tract after ingestion of the composition by a subject.

Hence, in some embodiments according to the disclosure, the composition is in the form of a food wherein the food is a cereal-based product, a dairy product, a juice drink, or a fermented food.

Examples of fermented foods include fermented milk products (such as yoghurt, kefir or lassi), fermented dairy-free milk alternatives (such as coconut milk kefir), fermented cereal-based products (such as oats, oatmeal, maize, sorghum, wheat), fermented vegetables (such as sauerkraut, kimchi, or pickles), fermented legumes or soybeans (such as natto or tempeh) and fermented tea (such as kombucha).

In use, the at least one strain or the composition comprising the at least one strain according to the disclosure may be mixed with a liquid or solid carrier before administration. For example, one may mix the strain or the composition thereof with a carrier comprising one or more liquids chosen from water, milk, coconut water, fruit drinks and juices, milk substitutes (soya drink, oat drink, nut and other plant-based drinks), sparkling beverages or some other aqueous solvent or drink prior to intake. Similarly, the at least one strain or the composition thereof may be mixed with a carrier consisting of one or more foods. Suitable food carriers include oatmeal carrier, barley carrier, fermented or non-fermented dairy products such as yoghurts, ice creams, milkshakes, fruit juices, beverages, soups, breads, biscuits, pasta, breakfast cereals, dry food bars including health bars, plant-based foods such as soy products, spreads, baby food, infant nutrition, infant formula, breast milk replacements from birth.

Another embodiment of the present disclosure relates to the method, the at least one strain or the use as described herein, wherein the carrier material is a food.

Yet, another embodiment of the present disclosure relates to the method, the at least one strain or the use as described herein, wherein the at least one strain of *Lactiplantibacillus plantarum* and/or *Lacticaseibacillus paracasei* is provided in a form selected from a solution, suspension, emulsion, tablet, granule, powder, capsule, lozenge, chewing gum, suppository, fast melt, or powder shot, preferably wherein the composition is provided in a capsule, fast melt or powder shot. Further, in a preferred embodiment of the present disclosure the composition is provided in the form of capsule.

The at least one strain may be administered in the form of a powdered composition such as a fast-melt microbial composition, for example those described in WO 2017/060477, or in Probi's UK Patent Application 1708932.7 or Probi's publication WO 2018/224509 relating to Probi^{®} Fast Melt technology. Where the powder is not in a fast-melt microbial composition, it may be suitable for being added to a food (e.g. yoghurt) or drink (e.g. water or milk) before ingestion.

Where the at least one strain is in the form of a powder, it would typically be filled in a sealed container, which provides an oxygen and moisture barrier in order to protect and maintain the viability of the bacteria in the composition. Hence, where the at least one strain is in the form of a powder, preferably the composition is packaged in sealed aluminium foil sticks, where each stick comprises one dose of the composition, *i.e.* one dose of the bacteria. Non-limiting examples of suitable containers include a stick, bag, pouch or capsule. In a preferred embodiment, the container is an aluminium foil or a polyethylene stick, which is typically sealed by welding. The stick is typically configured for easy tear opening. The stick may have a tear notch.

The at least one strain according to the disclosure may be formulated as a controlled-release solid dosage form, for example any of those described in WO 03/026687 and US Patent Nos. 8,007,777 and 8,540,980. The composition may be formulated as a layered dosage form, for example Probi's BIO*-tract^{®}* technology including any of the layered dosage forms described in WO 2016/003870.

A tablet may be made by compression or moulding, optionally with one or more accessory ingredients. Compressed tablets may be prepared by compressing in a suitable machine the at least one strain (e.g. freeze-dried) in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

A further embodiment of the present disclosure relates to a composition comprising at least one strain of *Lactiplantibacillus plantarum* and/or *Lacticaseibacillus paracasei* for use as defined by the present disclosure as described herein.

Yet, further embodiment of the present disclosure relates to a pharmaceutical composition comprising at least one strain of *Lactiplantibacillus plantarum* and/or *Lacticaseibacillus paracasei* for use as defined by the present disclosure as described herein.

Still, a further embodiment of the present disclosure relates to the pharmaceutical composition for use in reducing and/or preventing systemic inflammation in a subject in need thereof, preferably by formation of biofilm in the gastrointestinal tract.

Another embodiment of the present disclosure relates to a method comprising administering at least one strain of *Lactiplantibacillus plantarum* and/or *Lacticaseibacillus paracasei* substantially as described herein.

Yet, another embodiment of the present disclosure relates to the at least one strain of *Lactiplantibacillus plantarum* and/or *Lacticaseibacillus paracasei* for use substantially as described herein.

Still, another embodiment of the present disclosure relates to the use of at least one strain of *Lactiplantibacillus plantarum* and/or *Lacticaseibacillus paracasei* substantially as described herein.

A further, embodiment of the present disclosure relates to the use of at least one strain of *Lactiplantibacillus plantarum* and/or *Lacticaseibacillus paracasei* in the manufacture of a medicament for use as substantially as described herein.

Another embodiment of the present disclosure relates to a method of increasing or promoting biofilm formation of at least one strain of *Lactiplantibacillus plantarum* and/or *Lacticaseibacillus paracasei,* said method comprises the steps of:
- providing at least one strain of *Lactiplantibacillus plantarum* and/or *Lacticaseibacillus paracasei*
- subjecting said at least one strain of *Lactiplantibacillus plantarum* and/or *Lacticaseibacillus paracasei* to one or more pro-inflammatory cytokines are selected from the group consisting of interleukin-1β (IL-1β), interleukin-6 (IL-6), chemokine CXCL-8, interferon-gamma (IFN-γ), tumor necrosis factor (TNF-α) and any combination thereof, and
- obtaining (increased) biofilm formation in the at least one strain of *Lactiplantibacillus plantarum* and/or *Lacticaseibacillus paracasei.*

Biofilm formation is understood to be increased if the amount of biofilm formed by *Lactiplantibacillus plantarum* and/or *Lacticaseibacillus paracasei* subjected to one or more pro-inflammatory cytokines is greater than the amount of biofilm formed by *Lactiplantibacillus plantarum* and/or *Lacticaseibacillus paracasei* that has not been subjected to the same one or more pro-inflammatory cytokines.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising", "comprises", "preventing", "reducing", "supporting", "maintaining", "increasing", "promoting", "assisting", "provide", "providing", "treating", "improving" are to be construed as open-ended terms (i.e., meaning "including, but not limited to,") unless otherwise noted. Recitation of ranges of values herein are merely intended to serve as a shorth and method for referring individually to each separate value falling within the range, unless otherwise indicated herein, and each separate value is incorporated into the specification as if it were individually recited herein. All processes and methods described herein can be performed in any suitable order unless otherwise indicated herein or otherwise clearly contradicted by context. The use of any and all examples, or exemplary language (e.g., "such as" and i.e.) provided herein, is intended merely to better illuminate the disclosure and does not pose a limitation on the scope of the disclosure unless otherwise claimed. No language in the specification should be construed as indicating any non-claimed element as essential to the practice of the disclosure.

The listing or discussion of an apparently prior published document in this specification should not necessarily be taken as an acknowledgement that the document is part of the state of the art or is common general knowledge.

Preferences, options and embodiments for a given aspect, feature or parameter of the disclosure should, unless the context indicates otherwise, be regarded as having been disclosed in combination with any and all preferences, options and embodiments for all other aspects, features and parameters of the disclosure.

Certain embodiments and features of the present disclosure are also outlined in the following numbered items.

### REFERENCES

1. Adlerberth et al, 1996, Appl Environ Microbiol, 62(7):2244-2251
2. Hill et al, Nat Rev Gastroenterol Hepatol, 2014, 11(8):506-514
3. The Science and Practice of Pharmacy, 19th ed., vol. 1 & 2 (ed. Gennaro, 1995, Mack Publishing Company

### ITEMS

A1. A method for increasing and/or promoting biofilm formation in the gastrointestinal tract of a subject, comprising administering to a subject in need thereof, an effective dose of at least one strain of *Lactiplantibacillus plantarum* and/or *Lacticaseibacillus paracasei.*

A2. At least one strain of *Lactiplantibacillus plantarum* and/or *Lacticaseibacillus paracasei* for use in increasing and/or promoting biofilm formation in the gastrointestinal tract of a subject.

A3. Use of at least one strain of *Lactiplantibacillus plantarum* and/or *Lacticaseibacillus paracasei* for increasing and/or promoting biofilm formation in the gastrointestinal tract of a subject.

A4. Non-therapeutic use of at least one strain of *Lactiplantibacillus plantarum* and/or *Lacticaseibacillus paracasei* for increasing and/or promoting biofilm formation in the gastrointestinal tract of a subject.

A5. Use of at least one strain of *Lactiplantibacillus plantarum* and/or *Lacticaseibacillus paracasei* in the manufacture of a medicament for increasing and/or promoting biofilm formation in the gastrointestinal tract of a subject.

A6. Use of at least one strain of *Lactiplantibacillus plantarum* and/or *Lacticaseibacillus paracasei* in the manufacture of a supplement for increasing and/or promoting biofilm formation in the gastrointestinal tract of a subject.

A7. A method for reducing, preventing and/or treating inflammation in the gastrointestinal tract of a subject, comprising administering to a subject in need thereof, an effective dose of at least one strain of *Lactiplantibacillus plantarum* and/or *Lacticaseibacillus paracasei.*

A8. At least one strain of *Lactiplantibacillus plantarum* and/or *Lacticaseibacillus paracasei* for use in reducing, preventing and/or treating inflammation in the gastrointestinal tract of a subject.

A9. Use of at least one strain of *Lactiplantibacillus plantarum* and/or *Lacticaseibacillus paracasei* for reducing, preventing and/or treating inflammation in the gastrointestinal tract of a subject.

A10. Use of at least one strain of *Lactiplantibacillus plantarum* and/or *Lacticaseibacillus paracasei* in the manufacture of a medicament for reducing, preventing and/or treating inflammation in the gastrointestinal tract of a subject.

A11. A method for reducing, preventing and/or treating intestinal barrier dysfunction in a subject, comprising administering to a subject in need thereof, an effective dose of at least one strain of *Lactiplantibacillus plantarum* and/or *Lacticaseibacillus paracasei.*

A12. At least one strain of *Lactiplantibacillus plantarum* and/or *Lacticaseibacillus paracasei* for use in reducing, preventing and/or treating intestinal barrier dysfunction in a subject.

A13. Use of at least one strain of *Lactiplantibacillus plantarum* and/or *Lacticaseibacillus paracasei* for reducing, preventing and/or treating intestinal barrier dysfunction in a subject.

A14. Use of at least one strain of *Lactiplantibacillus plantarum* and/or *Lacticaseibacillus paracasei* in the manufacture of a medicament for reducing, preventing and/or treating intestinal barrier dysfunction in a subject.

A15. A method for improving and/or supporting gastrointestinal health in a subject, comprising administering to a subject in need thereof, an effective dose of at least one strain of *Lactiplantibacillus plantarum* and/or *Lacticaseibacillus paracasei.*

A16. At least one strain of *Lactiplantibacillus plantarum* and/or *Lacticaseibacillus paracasei* for improving and/or supporting gastrointestinal health in a subject.

A17. Use of at least one strain of *Lactiplantibacillus plantarum* and/or *Lacticaseibacillus paracasei* for improving and/or supporting gastrointestinal health in a subject.

A18. Use of at least one strain of *Lactiplantibacillus plantarum* and/or *Lacticaseibacillus paracasei* in the manufacture of a medicament for improving and/or supporting gastrointestinal health in a subject.

A19. A method for improving and/or supporting gastrointestinal homeostasis in a subject, comprising administering to a subject in need thereof, an effective dose of at least one strain of *Lactiplantibacillus plantarum* and/or *Lacticaseibacillus paracasei.*

A20. At least one strain of *Lactiplantibacillus plantarum* and/or *Lacticaseibacillus paracasei* for use in improving and/or supporting gastrointestinal homeostasis in a subject.

A21. Use of at least one strain of *Lactiplantibacillus plantarum* and/or *Lacticaseibacillus paracasei* for improving and/or supporting gastrointestinal homeostasis in a subject.

A22. Use of at least one strain of *Lactiplantibacillus plantarum* and/or *Lacticaseibacillus paracasei* in the manufacture of a medicament for improving and/or supporting gastrointestinal homeostasis in a subject.

A23. A method for supporting and/or maintaining gastrointestinal endothelial function in a subject, comprising administering to a subject in need thereof, an effective dose of at least one strain of *Lactiplantibacillus plantarum* and/or *Lacticaseibacillus paracasei.*

A24. At least one strain of *Lactiplantibacillus plantarum* and/or *Lacticaseibacillus paracasei* for use in supporting and/or maintaining gastrointestinal endothelial function in a subject.

A25. Use of at least one strain of *Lactiplantibacillus plantarum* and/or *Lacticaseibacillus paracasei* for supporting and/or maintaining gastrointestinal endothelial function in a subject.

A26. Use of at least one strain of *Lactiplantibacillus plantarum* and/or *Lacticaseibacillus paracasei* in the manufacture of a medicament for supporting and/or maintaining gastrointestinal endothelial function in a subject.

A27. A method for improving and/or supporting metabolic health in a subject, comprising administering to a subject in need thereof, an effective dose of at least one strain of *Lactiplantibacillus plantarum* and/or *Lacticaseibacillus paracasei.*

A28. At least one strain of *Lactiplantibacillus plantarum* and/or *Lacticaseibacillus paracasei* for use in improving and/or supporting metabolic health in a subject.

A29. Use of at least one strain of *Lactiplantibacillus plantarum* and/or *Lacticaseibacillus paracasei* for improving and/or supporting metabolic health in a subject.

A30. Use of at least one strain of *Lactiplantibacillus plantarum* and/or *Lacticaseibacillus paracasei* in the manufacture of a medicament for improving and/or supporting metabolic health in a subject.

A31. A method preventing and/or treating a gastrointestinal condition, a gastrointestinal disorder relating to GI health or to metabolic syndrome and/or a metabolic disease in a subject, comprising administering to a subject in need thereof, an effective dose of at least one strain of *Lactiplantibacillus plantarum* and/or *Lacticaseibacillus paracasei.*

A32. At least one strain of *Lactiplantibacillus plantarum* and/or *Lacticaseibacillus paracasei* for use in preventing and/or treating a gastrointestinal condition, a gastrointestinal disorder relating to GI health or to metabolic syndrome and/or a metabolic disease in a subject.

A33. Use of at least one strain of *Lactiplantibacillus plantarum* and/or *Lacticaseibacillus paracasei* for preventing and/or treating a gastrointestinal condition, a gastrointestinal disorder relating to GI health or to metabolic syndrome and/or a metabolic disease in a subject.

A34. Use of at least one strain of *Lactiplantibacillus plantarum* and/or *Lacticaseibacillus paracasei* in the manufacture of a medicament for preventing and/or treating a gastrointestinal condition, a gastrointestinal disorder relating to GI health or to metabolic syndrome and/or a metabolic disease in subject.

A35. A method for reducing, preventing and/or treating systemic inflammation in a subject, comprising administering to a subject in need thereof, an effective dose of at least one strain of *Lactiplantibacillus plantarum* and/or *Lacticaseibacillus paracasei.*

A36. At least one strain of *Lactiplantibacillus plantarum* and/or *Lacticaseibacillus paracasei* for use in reducing, preventing and/or treating systemic inflammation in a subject.

A37. Use of at least one strain of *Lactiplantibacillus plantarum* and/or *Lacticaseibacillus paracasei* for reducing, preventing and/or treating systemic inflammation in a subject.

A38. Use of at least one strain of *Lactiplantibacillus plantarum* and/or *Lacticaseibacillus paracasei* in the manufacture of a medicament reducing, preventing and/or treating systemic inflammation in a subject.

A39. A method for increasing growth of at least one strain of *Lactiplantibacillus plantarum* in the gastrointestinal tract of a subject having an elevated expression of one or more pro-inflammatory cytokines, comprising administering to a subject in need thereof, an effective dose of at least one strain of *Lactiplantibacillus plantarum.*

A40. At least one strain of *Lactiplantibacillus plantarum* for use in increasing growth of at least one strain of *Lactiplantibacillus plantarum* in the gastrointestinal tract of a subject having an elevated expression of one or more pro-inflammatory cytokines.

A41. Use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A42. Non-therapeutic use of at least one strain of *Lactiplantibacillus plantarum* for increasing growth of at least one strain of *Lactiplantibacillus plantarum* in the gastrointestinal tract of a subject having an elevated expression of one or more pro-inflammatory cytokines.

A43. Use of at least one strain of *Lactiplantibacillus plantarum* in the manufacture of a medicament for increasing growth of at least one strain of Lactiplantibacillus plantarum in the gastrointestinal tract of a subject having an elevated expression of one or more pro-inflammatory cytokines.

A44. Use of at least one strain of Lactiplantibacillus plantarum in the manufacture of a supplement for increasing growth of at least one strain of *Lactiplantibacillus plantarum* in the gastrointestinal tract of a subject having an elevated expression of one or more pro-inflammatory cytokines.

A45. The method according to any one of items A1, A7, A11, A15, A19, A23, A27, A31, A35 or A39, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the biofilm comprises *Lactiplantibacillus plantarum* and/or *Lacticaseibacillus paracasei.*

A46. The method according to items A1, A7, A11, A15, A19, A23, A27, A31, A35, A39 or 45, the at least one strain according to items A2, A8, A12, A16, A20, A24, A28, A32, A36 or A40 or the use according to any one of items A3 to A6, A9, A10, A13, A14, A17, A18, A21, A22, A25, A26, A29, A30, A33, A34, A37, A38 or A41 to A44, wherein the biofilm is formed in the gastrointestinal tract after at least two weeks of administration of the at least one strain of *Lactiplantibacillus plantarum* and/or *Lacticaseibacillus paracasei* to the subject.

A47. The method according to any one of items A1, A7, A11, A15, A19, A23, A27, A31, A35, A39 or 45 to 46, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the subject has an elevated level of pro-inflammatory cytokines in a body fluid selected from the group consisting of blood, saliva, feces, intestinal tissue and combinations thereof.

A48. The method according to any one of items A1, A7, A11, A15, A19, A23, A27, A31, A35, A39 or 45 to 47, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38, A41 to A44, wherein the subject has an elevated expression of one or more pro-inflammatory cytokine(s) in intestinal cells or in the intestine.

A49. The method according to any one of items A1, A7, A11, A15, A19, A23, A27, A31, A35, A39 or 45 to 48, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the one or more pro-inflammatory cytokines are selected from the group consisting of interleukin-1β (IL-1β), interleukin-6 (IL-6), chemokine (CXCL-8), interferon-gamma (IFN-γ), tumor necrosis factor (TNF-α) and any combination thereof.

A50. The method according to any one of items A1, A7, A11, A15, A19, A23, A27, A31, A35, A39 or 45 to 49, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the pro-inflammatory cytokine is interleukin-1β (IL-1β).

A51. The method according to any one of items A1, A7, A11, A15, A19, A23, A27, A31, A35, A39 or 45 to 50, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the subject is a mammal, preferably wherein the subject is human.

A52. The method according to any one of items A1, A7, A11, A15, A19, A23, A27, A31, A35, A39 or 45 to 51, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the disorder is a gastrointestinal disorder and wherein the gastrointestinal disorder is selected from the group consisting of irritable bowel syndrome (IBS), inflammatory bowel disease (IBD), or any of the symptoms often included in these disorders; abdominal pain, flatulence, bloating, constipation, diarrhea and defecation disability.

A53. The method according to any one of items A1, A7, A11, A15, A19, A23, A27, A31, A35, A39 or 45 to 52, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein inflammatory bowel disease (IBD) is selected from Crohn's disease, ulcerative colitis, microscopic colitis and any combination thereof.

A54. The method according to any one of items A1, A7, A11, A15, A19, A23, A27, A31, A35, A39 or 45 to 53, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the condition is a dysbiosis and wherein the dysbiosis is a gastrointestinal dysbiosis.

A55. The method according to any one of items A1, A7, A11, A15, A19, A23, A27, A31, A35, A39 or 45 to 54, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the gastrointestinal dysbiosis is a microbial dysbiosis in the gastrointestinal tract.

A56. The method according to any one of items A1, A7, A11, A15, A19, A23, A27, A31, A35, A39 or 45 to 55, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the systemic inflammation is associated with one or more disease(s), one or more disorder(s), one or more syndrome(s) and/or one or more condition(s).

A57. The method according to any one of items A1, A7, A11, A15, A19, A23, A27, A31, A35, A39 or 45 to 56, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the disease is a metabolic disease and wherein the metabolic disease is selected from the group consisting of type 2 diabetes mellitus, obesity, atherosclerosis and cardiovascular disease.

A58. The method according to any one of items A1, A7, A11, A15, A19, A23, A27, A31, A35, A39 or 45 to 57, the at least one strain according to any one of items A2, A8, A12, A16, A20, A24, A28, A32, A36 or A40 or the use according to any one of items A3 to A6, A9, A10, A13, A14, A17, A18, A21, A22, A25, A26, A29, A30, A33, A34, A37, A38 or A41 to A44, wherein the syndrome is metabolic syndrome and wherein the metabolic syndrome is selected from the group consisting of increased blood pressure, high blood sugar level, excess body fat around the waist and abnormal cholesterol or triglyceride levels.

A59. The method according to any one of items A1, A7, A11, A15, A19, A23, A27, A31, A35, A39 or 45 to 58, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* can adhere to the intestinal epithelium and persist in the intestine.

A60. The method according to any one of items A1, A7, A11, A15, A19, A23, A27, A31, A35, A39 or 45 to 59, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* comprises a mannose-specific adhesin.

A61. The method according to any one of items A1, A7, A11, A15, A19, A23, A27, A31, A35, A39 or 45 to 60, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* is selected from *Lactiplantibacillus plantarum* DSM 6595 (299), *Lactiplantibacillus plantarum* DSM 9843 (299v), *Lactiplantibacillus plantarum* DSM 15312 (HEAL 9), *Lactiplantibacillus plantarum* DSM 15313 (HEAL 19), *Lactiplantibacillus plantarum* DSM 15316 (HEAL 99), *Lactiplantibacillus plantarum* DSM 32131 (GOS42), *Lactiplantibacillus plantarum* DSM 17852 (LB3e) and *Lactiplantibacillus plantarum* DSM 17853 (LB7c).

A62. The method according to any one of items A1, A7, A11, A15, A19, A23, A27, A31, A35, A39 or 45 to 61, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* is selected from *Lactiplantibacillus plantarum* DSM 6595 (299), *Lactiplantibacillus plantarum* DSM 9843 (299v), *Lactiplantibacillus plantarum* DSM 15312 (HEAL 9) and any combination thereof.

A63. The method according to any one of items A1, A7, A11, A15, A19, A23, A27, A31, A35, A39 or 45 to 62, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* is selected from *Lactiplantibacillus plantarum* DSM 9843 (299v), *Lactiplantibacillus plantarum DSM* 15312 (HEAL 9) or the combination of *Lactiplantibacillus plantarum* DSM 9843 (299v) and *Lactiplantibacillus plantarum* DSM 15312 (HEAL 9).

A64. The method according to any one of items A1, A7, A11, A15, A19, A23, A27, A31, A35, A39 or 45 to 63, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* is *Lactiplantibacillus plantarum* DSM 9843 (299v).

A65. The method according to any one of items A1, A7, A11, A15, A19, A23, A27, A31, A35, A39 or 45 to 64, the at least one strain according to any one of items A2, A8, A12, A16, A20, A24, A28, A32, A36, A40 or the use according to any one of items A3 to A6, A9, A10, A13, A14, A17, A18, A21, A22, A25, A26, A29, A30, A33, A34, A37, A38, A41 to A44, wherein the at least one strain of *Lacticaseibacillus paracasei* is selected from *Lacticaseibacillus paracasei* DSM 13434 (8700:2) or *Lacticaseibacillus paracasei* DSM 13432 (02A).

A66. The method according to any one of items A1, A7, A11, A15, A19, A23, A27, A31, A35, A39 or 45 to 65, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lacticaseibacillus paracasei* is *Lacticaseibacillus paracasei* DSM 13434 (8700:2).

A67. The method according to any one of items A1, A7, A11, A15, A19, A23, A27, A31, A35, A39 or 45 to 66, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain is administered at a daily dose of from about 1 x 10^6 to about 1 x 10^14 colony forming units (CFU) and/or about 1 x 10^9 to about 1 x 10^11 CFU.

A68. The method according to any one of items A1, A7, A11, A15, A19, A23, A27, A31, A35, A39 or 45 to 67, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the daily dose of the at least one strain is 1 x 10^10 CFU.

A69. The method according to any one of items A1, A7, A11, A15, A19, A23, A27, A31, A35, A39 or 45 to 68, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain is administered daily and/or in a single unit dosage form.

A70. The method according to any one of items A1, A7, A11, A15, A19, A23, A27, A31, A35, A39 or 45 to 69, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain is administered daily for at least one week, such as for at least two weeks, e.g. for at least three weeks, such as at least four weeks, e.g. for at least five weeks, such as at least six weeks, e.g. at least seven weeks, at least eight weeks, e.g. at least nine weeks, at least ten weeks, such as at least eleven weeks or at least twelve weeks.

A71. The method according to any one of items A1, A7, A11, A15, A19, A23, A27, A31, A35, A39 or 45 to 70, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain is administered daily for at least two weeks.

A72. The method according to any one of items A1, A7, A11, A15, A19, A23, A27, A31, A35, A39 or 45 to 71, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* and/or *Lacticaseibacillus paracasei* is alive, dead or attenuated (e.g. a postbiotic and/or paraprobiotic and/or heat-inactivated), preferably wherein the at least one strain is alive.

A73. The method according to any one of items A1, A7, A11, A15, A19, A23, A27, A31, A35, A39 or 45 to 72, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain is administered in the form of a composition comprising at least one pharmaceutically and/or nutritionally acceptable carrier, excipient and/or diluent material.

A74. The method according to any one of items A1, A7, A11, A15, A19, A23, A27, A31, A35, A39 or 45 to 73, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the carrier material is a food.

A75. The method according to any one of items A1, A7, A11, A15, A19, A23, A27, A31, A35, A39 or 45 to 74, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the at least one strain of *Lactiplantibacillus plantarum* and/or *Lacticaseibacillus paracasei* is provided in a form selected from a solution, suspension, emulsion, tablet, granule, powder, capsule, lozenge, chewing gum, suppository, fast melt, or powder shot, preferably wherein the composition is provided in a capsule, fast melt or powder shot.

A76. The method according to any one of items A1, A7, A11, A15, A19, A23, A27, A31, A35, A39 or 45 to 75, the at least one strain according to any one of items A2, A8, A12, A16, A20, A24, A28, A32, A36 or A40, or the use according to any one of items A3 to A6, A9, A10, A13, A14, A17, A18, A21, A22, A25, A26, A29, A30, A33, A34, A37, A38 or A41 to A44, wherein the composition is provided in the form of a capsule.

B1. A composition comprising at least one strain of *Lactiplantibacillus plantarum* and/or *Lacticaseibacillus paracasei* for use as defined in any one of items A1-A76.

C1. A pharmaceutical composition comprising at least one strain of *Lactiplantibacillus plantarum* and/or *Lacticaseibacillus paracasei* for use as defined in any one of items A1-A76.

C2. The pharmaceutical composition according to item C1 for use in reducing and/or preventing systemic inflammation in a subject in need thereof, preferably by formation of biofilm in the gastrointestinal tract.

D1. A method comprising administering at least one strain of *Lactiplantibacillus plantarum* and/or *Lacticaseibacillus paracasei* substantially as described herein.

D2. At least one strain of *Lactiplantibacillus plantarum* and/or *Lacticaseibacillus paracasei* for use substantially as described herein.

D3. Use of at least one strain of *Lactiplantibacillus plantarum* and/or *Lacticaseibacillus paracasei* substantially as described herein.

D4. Use of at least one strain of *Lactiplantibacillus plantarum* and/or *Lacticaseibacillus paracasei* in the manufacture of a medicament for use as substantially as described herein.

E1. A method of increasing or promoting biofilm formation of at least one strain of *Lactiplantibacillus plantarum* and/or *Lacticaseibacillus paracasei,* said method comprises the steps of:
- providing at least one strain of *Lactiplantibacillus plantarum* and/or *Lacticaseibacillus paracasei*
- subjecting said at least one strain of *Lactiplantibacillus plantarum* and/or *Lacticaseibacillus paracasei* to one or more pro-inflammatory cytokines are selected from the group consisting of interleukin-1β (IL-1β), interleukin-6 (IL-6), chemokine CXCL-8, interferon-gamma (IFN-γ), tumor necrosis factor (TNF-α) and any combination thereof, and
- obtaining (increased) biofilm formation in the at least one strain of *Lactiplantibacillus plantarum* and/or *Lacticaseibacillus paracasei.*

### EXAMPLES

### Example 1 - Effects of pro-inflammatory cytokines on the growth and biofilm formation of probiotic strains

The purpose of this study was to investigate the effects of pro-inflammatory cytokines IFN-γ, IL-6, TNF-α, IL-1β, and CXCL8 on the growth and biofilm formation of three probiotic strains *Lactiplantibacillus plantarum* DSM 9843, *Lactiplantibacillus plantarum* DSM 15312, and *Lacticaseibacillus paracasei* DSM 13434. An additional element of the study was to study the effect of TNF-α on the growth and biofilm formation of *Escherichia coli (E. coli) ATCC 8739.*

### Methods and Materials

### Growth Assay

*Lactiplantibacillus plantarum* DSM 9843, *Lactiplantibacillus plantarum* DSM 15312 and *Lacticaseibacillus paracasei* DSM 13434 were cultured in 5mL of De Man, Rogosa and Sharpe (MRS) broth (Fisher Scientific, Merelbeke, Belgium) at 37°C and on an orbital shaker at 200rpm for 24 hours. *Escherichia coli (E. coli) ATCC 8739* was cultured in 5mL of Luria-Bertani (LB) broth (Difco Laboratories, Detroit, MI, USA) at 37°C and on an orbital shaker at 200rpm for 16 hours. After incubation, microbial cultures were centrifuged at 6000rpm for 10 minutes, the supernatant was discarded, and microbial cells were resuspended in 1mL of minimal salt solution (MSS, 1.3% [w/v] Na2HPO4, 0.3% KH2PO4, 0.05% NaCl, and 0.1% NH4Cl supplemented with 20mM glucose, 2mM MgSO4, 100µM CaCl2, and 0.25% casamino acids). 4µL of the resuspended *Lactiplantibacillus plantarum* DSM 9843, *Lactiplantibacillus plantarum* DSM 15312 and *Lacticaseibacillus paracasei* DSM 13434 were diluted in a new Eppendorf tube with 996µL of MRS, so as to achieve an optical density (OD) of approximately 0.120 at 2 hours; 4µL of the *E. coli* were diluted in 996µL MSS for an OD around 0.040, at 2 hours. Using a 96-well plate, TNF-α (T6674), IL-1β (I9401), CXCL8 (I1645), IFN-γ (SRP3058) or IL-6 (I1395) (Sigma-Aldrich, St. Louis, MO, United States) were individually tested at a concentration of 40ng/mL, by being directly added into the well containing 95µL of medium and 5µL of bacteria. There were no other additives. The effect on the growth was measured in real-time for up to 24 hours, with a time interval equal to five minutes. Growth was quantified in relation to the OD at 600nm with the use of a Spectrophotometer (Cytation 3, Biotek Inc, Winooski, VT, USA).

### Biofilm formation Assay

Like the growth assay, after a 24-hour incubation with the cytokines, the microtiter plate was washed three times using sterile ddH2O and then it was left to dry for 45 minutes. Each well was stained with 175µL of crystal violet (CV) 0.1% dye for 20 minutes at room temperature. After repeating the washing step with ddH2O, the plate was left to dry for three hours. Ethanol 95%, 200µL, was added into each of the wells and then 100µL were transferred into a new 96-well plate for biofilm formation quantification via optic density at 540nm (Cytation 3, Biotek Inc, Winooski, VT, USA). The biofilm was then calculated in relation to the control, the un-treated bacteria.

### Statistical Analyses

Data were analyzed in Microsoft^{®} 365 Excel^{®} Version 2203 (2022). For growth and biofilm production, the comparison between each of the different cytokine incubations (TNF-α/IL-1β/IFN-γ/IL-6/CXCL8) and the control was conducted using paired, two-tailed, Student's T-test with p-values≤0.05 corresponding to statistically significant results (a=5%). Benjamini-Yekutieli's test was used as a correction for false-discovery rate (FDR) on the growth measurements.

### Results

### Cytokines induce increased growth in specific strains at specific timepoints

The growth was evaluated for *Lactiplantibacillus plantarum* DSM 9843, *Lactiplantibacillus plantarum* DSM 15312, *Lacticaseibacillus paracasei* DSM 13434and the *E. coli* strain. Measurements were taken for 24 hours and for the analysis the focus was on 0, 8, 12, 16, and 24 hours for the *Lactobacilli,* while 0, 2, 4, 6, 8, and 24 were selected for the *E. coli* (Figure 1). After incubation with TNF-α at 40ng/mL, growth was not affected in neither the *E. coli* strain (Figure 1D), nor the probiotics *Lacticaseibacillus paracasei* DSM 13434 and *Lactiplantibacillus plantarum* DSM 9843 (Figure 1A, C). A statistically significant increase by TNF-α was observed at 8 hours (start of log-phase) only for *Lactiplantibacillus plantarum* DSM 15312 (Figure 1B). Generally, none of the tested cytokines influenced the growth of *Lacticaseibacillus paracasei* DSM 13434 (Figure 1A), however IFN-γ, IL-6, IL-1β, and CXCL8 induced statistically significant increases of growth in the *Lactiplantibacillus plantarum* DSM 15312 and *Lactiplantibacillus plantarum* DSM 9843 strains (Figure 1B-C). IFN-γ significantly increased growth at 8, 12, 16 and 24 hours for both *Lactiplantibacillus plantarum* DSM 15312 and *Lactiplantibacillus plantarum* DSM 9843, while IL-6 caused an increase at 8 and 12 hours for *Lactiplantibacillus plantarum* DSM 15312 and at 8, 12 and 16 hours for *Lactiplantibacillus plantarum* DSM 9843. IL-1β only increased growth for *Lactiplantibacillus plantarum* DSM 15312 at 8 hours and lastly, CXCL8 caused a positive effect only at 8 hours for *Lactiplantibacillus plantarum* DSM 15312, and at 8 and 12 hours for *Lactiplantibacillus plantarum* DSM 9843.

### Increased biofilm formation in the probiotic strains

Production of biofilm was assessed across the three probiotic strains *Lactiplantibacillus plantarum* DSM 9843, *Lactiplantibacillus plantarum* DSM 15312 and *Lacticaseibacillus paracasei* DSM 13434. *Lactiplantibacillus plantarum* DSM 9843 was found to be the most sensitive strain to cytokine stimulation, as its capacity for biofilm formation was significantly increased by all the pro-inflammatory cytokines evaluated. IL-1β induced the largest effect by causing an increase of 34.2±10.6%, followed by TNF-α (28.4±7.4%), then IFN-γ (26.6±8.4%), CXCL8 (21.8±11.1%), and lastly IL-6 (15.9±9.4%) in comparison with the control (Figure 2C). The *Lacticaseibacillus paracasei* DSM 13434 and *Lactiplantibacillus plantarum* DSM 15312 strains were only affected by a single cytokine (IL-8β). *Lactiplantibacillus plantarum* DSM 15312 produced significantly more biofilm after incubation with IL-1β, presenting a 27.4±10.8% improvement (Figure 2B), while *Lacticaseibacillus paracasei* DSM 13434 presented a statistically significant increase of 8.6±5.3% from incubation with CXCL8 (Figure 2A). TNF-α had a significantly positive impact on the biofilm formation of *Lactiplantibacillus plantarum* DSM 9843, but it did not induce an effect on *E. coli* (Figure 2D).

### Discussion and Conclusion

In this study, the effects of five different pro-inflammatory cytokines TNF-α, IL-1β, IL-6, IFN-γ and CXCL8 were assessed on three probiotic strains *Lactiplantibacillus plantarum* DSM 9843, *Lactiplantibacillus plantarum* DSM 15312, *Lacticaseibacillus paracasei* DSM 13434 and *E. coli ATCC 8739* strain, in order to determine if any differences would occur regarding their growth and biofilm formation. *LacticaseibacillusThe* results show that *Lactiplantibacillus plantarum* DSM 9843, *Lactiplantibacillus plantarum* DSM 15312 and *Lacticaseibacillus paracasei* DSM 13434respond differently after the 24-hour incubation with the same cytokine suggesting a strain specific response to different cytokines. Although, more similar responses were displayed by *Lactiplantibacillus plantarum* DSM 15312 and *Lactiplantibacillus plantarum* DSM 9843, since they belong in the same genus *(Lactiplantibacillus),* compared to *Lacticaseibacillus paracasei* DSM 13434 *(Lacticaseibacillus).* In regard to growth, *Lacticaseibacillus paracasei* DSM 13434 seemed not to be stimulated by any of the cytokines tested, whereas *Lactiplantibacillus plantarum* DSM 15312 and *Lactiplantibacillus plantarum* DSM 9843 incubated with IFN-γ presented an increased growth, especially during the start of their stationary phase at 24-hours. However, in other phases, for instance at 12 hours (log-phase), CXCL8 caused an improvement of growth for *Lactiplantibacillus plantarum* DSM 9843, but not for *Lactiplantibacillus plantarum* DSM 15312. Concerning biofilm formation, the strains exhibited significant differences, with *Lacticaseibacillus paracasei* DSM 13434 showing an enhanced biofilm production after CXCL8 24-hour incubation, while the other two strains showed the highest increase by IL-1β. Interestingly, *Lactiplantibacillus plantarum* DSM 9843 biofilm production was significantly increased by all the evaluated cytokines, indicating that this strain is particularly sensitive to pro-inflammatory molecules.

Throughout the experiments, it was observed that incubation with specific cytokines led to the improvement of a specific function. For instance, IL-1β significantly elevated biofilm formation, but did not significantly improve growth for the strains *Lactiplantibacillus plantarum* DSM 15312 and *Lactiplantibacillus plantarum* DSM 9843. In this study, *Lactiplantibacillus plantarum* DSM 9843 presented the highest increase in biofilm formation, which could improve its already strong mannose-adhesion capacity on the mucosal epithelium. Additionally, the *Lactiplantibacillus plantarum* DSM 9843 strain has been described to possess a highly diverse functional genome, compared to other lactic acid bacterial strains, which might justify the higher sensitivity to the pro-inflammatory cytokines compared with the other two strains.

In conclusion, these results provide evidence of the capacity of pro-inflammatory cytokines to significantly increase the biofilm production of three different *Lactobacilli* strains, *Lactiplantibacillus plantarum* DSM 15312, *Lactiplantibacillus plantarum* DSM 9843 and *Lacticaseibacillus paracasei* DSM 13434 and even cause increases in growth of *Lactiplantibacillus plantarum* DSM 15312 and *Lactiplantibacillus plantarum* DSM 9843 strains. The changes induced by cytokine incubation, were observed to be strain specific, cytokine and time dependent and could potentially play a key role in the efficiency of the probiotic. IL-1β caused the most prominent effect on the biofilm of *Lactiplantibacillus plantarum* DSM 15312 and *Lactiplantibacillus plantarum* DSM 9843, with the *Lactiplantibacillus plantarum* DSM 9843 presenting significantly higher biofilm formation in response to all cytokine treatments.

The above findings suggest that *Lactiplantibacillus plantarum* DSM 15312, *Lactiplantibacillus plantarum* DSM 9843 and *Lacticaseibacillus paracasei* DSM 13434 can be a useful tool for preventing and reducing excessive immune responses in the gastrointestinal tract, and for supporting intestinal homeostasis in general.

## Claims

1. At least one strain of *Lactiplantibacillus plantarum* and/or *Lacticaseibacillus paracasei* for use in increasing and/or promoting biofilm formation in the gastrointestinal tract of a subject.

2. The at least one strain of *Lactiplantibacillus plantarum* and/or *Lacticaseibacillus paracasei* according to claim 1, wherein the subject has an elevated expression of one or more pro-inflammatory cytokines

3. The at least one strain of *Lactiplantibacillus plantarum* and/or *Lacticaseibacillus paracasei* according to claims 1 or 2, wherein the biofilm comprises *Lactiplantibacillus plantarum* and/or *Lacticaseibacillus paracasei* and/or is formed by *Lactiplantibacillus plantarum* and/or *Lacticaseibacillus paracasei*

4. The at least one strain of *Lactiplantibacillus plantarum* and/or *Lacticaseibacillus paracasei* according to any one of the preceding claims 2, wherein the biofilm is formed in gastrointestinal tract after at least two weeks of administration of the at least one strain of *Lactiplantibacillus plantarum* and/or *Lacticaseibacillus paracasei* to the subject

5. The at least one strain of *Lactiplantibacillus plantarum* and/or *Lacticaseibacillus paracasei* according to claim 2, wherein the elevated level of pro-inflammatory cytokines is in a body fluid selected from the group consisting of blood, saliva, feces, intestinal tissue and any combination thereof

6. The at least one strain of *Lactiplantibacillus plantarum* and/or *Lacticaseibacillus paracasei* according to any one of the preceding claims, wherein the subject has an elevated expression of one or more pro-inflammatory cytokine(s) in intestinal cells or in the intestine

7. The at least one strain of *Lactiplantibacillus plantarum* and/or *Lacticaseibacillus paracasei* according to any one of the preceding claims, wherein the one or more pro-inflammatory cytokines are selected from the group consisting of interleukin-1β (IL-1β), interleukin-6 (IL-6), interleukin-6 (IL-6)/chemokine (CXCL-8), interferon-gamma (IFN-γ), tumor necrosis factor (TNF-α) and any combination thereof

8. The at least one strain of *Lactiplantibacillus plantarum* and/or *Lacticaseibacillus paracasei* according to any one of the preceding claims, wherein the pro-inflammatory cytokine is interleukin-1β is (IL-1β)

9. The at least one strain of *Lactiplantibacillus plantarum* and/or *Lacticaseibacillus paracasei* according to any one of the preceding claims, wherein the subject is a mammal, preferably wherein the subject is human

10. The at least one strain of *Lactiplantibacillus plantarum* and/or *Lacticaseibacillus paracasei* according to any one of the preceding claims, wherein the at least one strain of *Lactiplantibacillus plantarum* is selected from *Lactiplantibacillus plantarum* DSM 6595 *(299), Lactiplantibacillus plantarum* DSM 9843 (299v), *Lactiplantibacillus plantarum* DSM 15312 (HEAL 9), *Lactiplantibacillus plantarum* DSM 15313 (HEAL 19), *Lactiplantibacillus plantarum* DSM 15316 (HEAL 99), *Lactiplantibacillus plantarum* DSM 32131 (GOS42), *Lactiplantibacillus plantarum* DSM 17852 (LB3e) and *Lactiplantibacillus plantarum* DSM 17853 (LB7c)

11. The at least one strain of *Lactiplantibacillus plantarum* and/or *Lacticaseibacillus paracasei* according to any one of of the preceding claims, wherein the at least one strain of *Lactiplantibacillus plantarum* is *Lactiplantibacillus plantarum* DSM 9843 (299v) and/or *Lactiplantibacillus plantarum* DSM 15312 (HEAL 9)

12. The at least one strain of *Lactiplantibacillus plantarum* and/or *Lacticaseibacillus paracasei* according to any one of the preceding claims, wherein the at least one strain of *Lacticaseibacillus paracasei* is *Lacticaseibacillus paracasei* DSM 13434 (8700:2)

13. The at least one strain of *Lactiplantibacillus plantarum* and/or *Lacticaseibacillus paracasei* according to any one of the preceding claims, wherein the at least one strain is administered at a daily dose of from about 1 x 10⁶ to about 1 x 10¹⁴ colony forming units (CFU) and/or about 1 x 10⁹ to about 1 x 10¹¹ CFU

14. The at least one strain of *Lactiplantibacillus plantarum* and/or *Lacticaseibacillus paracasei* according to any one of the preceding claims, wherein the at least one strain of *Lactiplantibacillus plantarum* and/or *Lacticaseibacillus paracasei* is alive, dead or attenuated (e.g. a postbiotics and/or paraprobiotic and/or heat-inactivated), preferably wherein the at least one stain is alive

15. A method of increasing or promoting biofilm formation of at least one strain of *Lactiplantibacillus plantarum* and/or *Lacticaseibacillus paracasei,* said method comprises the steps of:
- providing at least one strain of *Lactiplantibacillus plantarum* and/or *Lacticaseibacillus paracasei*
- subjecting said at least one strain of *Lactiplantibacillus plantarum* and/or *Lacticaseibacillus paracasei* to one or more pro-inflammatory cytokines are selected from the group consisting of interleukin-1β (IL-1β), interleukin-6 (IL-6), chemokine CXCL-8, interferon-gamma (IFN-γ), tumor necrosis factor (TNF-α) and any combination thereof, and
- obtaining biofilm formation in the at least one strain of *Lactiplantibacillus plantarum* and/or *Lacticaseibacillus paracasei*
